# EUROPEAN PATENT APPLICATION

(11) **EP 4 574 160 A1**
(43) Date of publication of application: **25.06.2025**
(21) Application number: 24221945.9
(22) Date of filing: 19.12.2024
(51) Int. Cl.: A61K 35/745, A23L 33/135, A61P 11/06, A61P 17/00, A61P 29/00, A61P 37/08

(54) **COMPOSITION COMPRISING BIFIDOBACTERIUM BREVE STRAIN FOR USE IN THE PREVENTION OF SKIN ALLERGIES**

(30) Priority: 20.12.2023 EP 23218777
(71) Applicant: Hipp & Co, 6072 Sachseln (CH)
(72) Inventor: Schaubeck, Monika, 85276 Paffenhofen (DE); Grube, Jana, 85276 Pfaffenhofen (DE); Bergougnan, Carolin, 85276 Pfaffenhofen (DE); Volz, Thomas, 80802 München (DE); Amar, Yacine, 80802 München (DE)
(74) Representative: Schiweck Weinzierl Koch Patentanwälte Partnerschaft mbB

(57) **Abstract**

The present invention relates to a synthetic composition, such as an infant formula or a weaning food, comprising the *Bifidobacterium breve* strain, which is deposited with the DSMZ under the accession number DSM 32583, for use in a method of preventing at least one IL-10 mediated disease by enhancing expression of IL-10 in a subject.

## Description

### FIELD OF THE INVENTION

The present invention relates to a synthetic composition, such as an infant formula or a weaning food, comprising the *Bifidobacterium breve* strain, which is deposited with the DSMZ under the accession number DSM 32583, for use in a method of preventing at least one IL-10 mediated disease by enhancing expression of IL-10 in a subject.

### BACKGROUND OF THE INVENTION

Among the intestinal commensals, members of the *Bifidobacteriaceae* and *Lactobacillaceae* families are dedicated a particular attention due to their numerous reported health benefits. Also they have been attributed preventive and curative roles in gastrointestinal infections, diarrhoea, inflammatory bowel diseases, colon cancer, allergies, obesity, diabetes, osteoporosis, and the like.

With respect to particularly human newborn infants, there is ongoing research about which particular strains of gut bacteria, including *Bifidobacterium* spp., are beneficial, e.g. for the newborn infant's health and to what extent. There is thus an ongoing need in the art to identify and provide novel gut bacteria that might be used in the formulation of compositions, such as infant formulae or weaning foods, which provide certain health benefits.

The technical problem underlying the present application is thus to provide a new gut bacteria strain which is suitable for the application in compositions providing health benefits, particularly to infants. The technical problem is solved by providing the embodiments reflected in the claims, described in the description, and illustrated in the examples and figures that follow.

### SUMMARY OF THE INVENTION

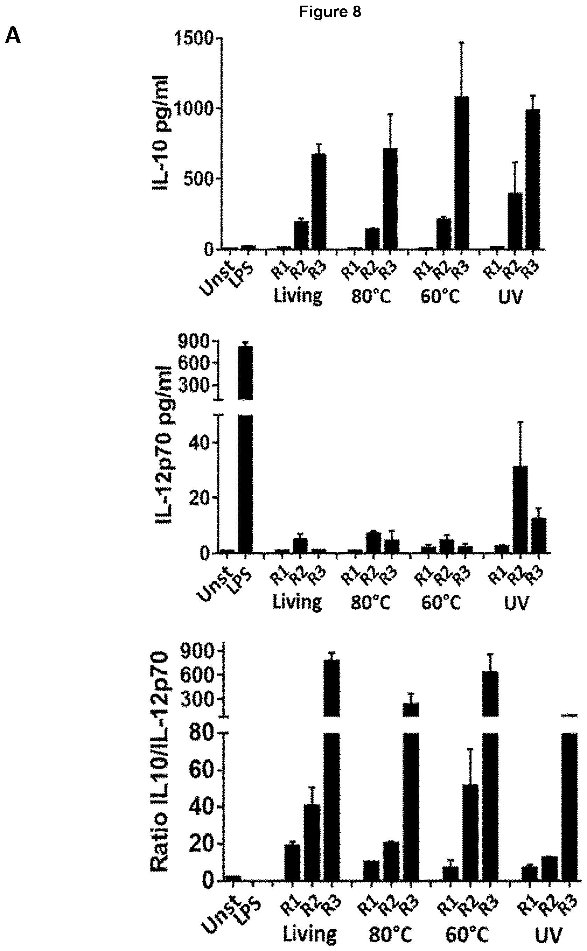

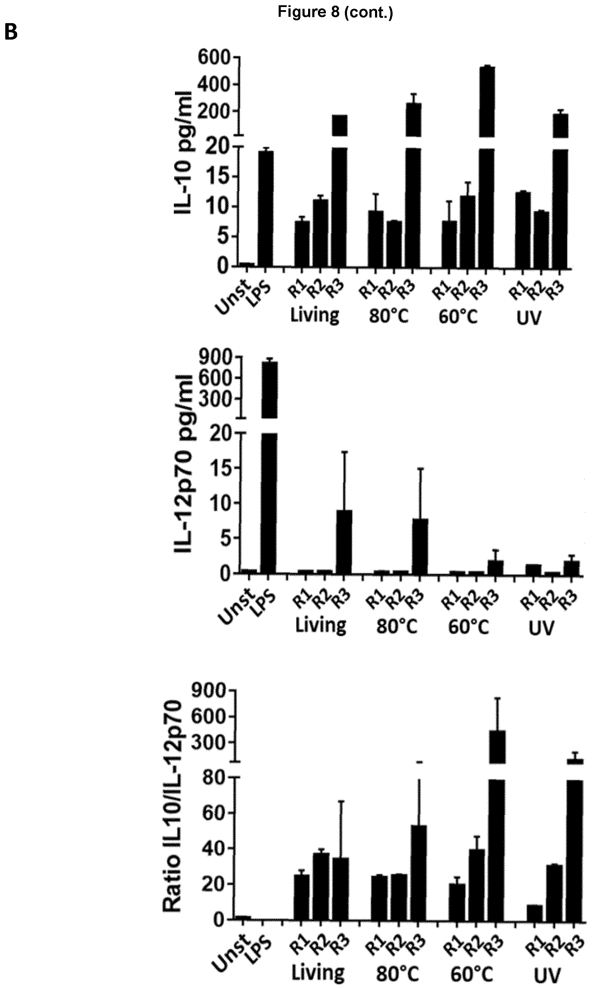

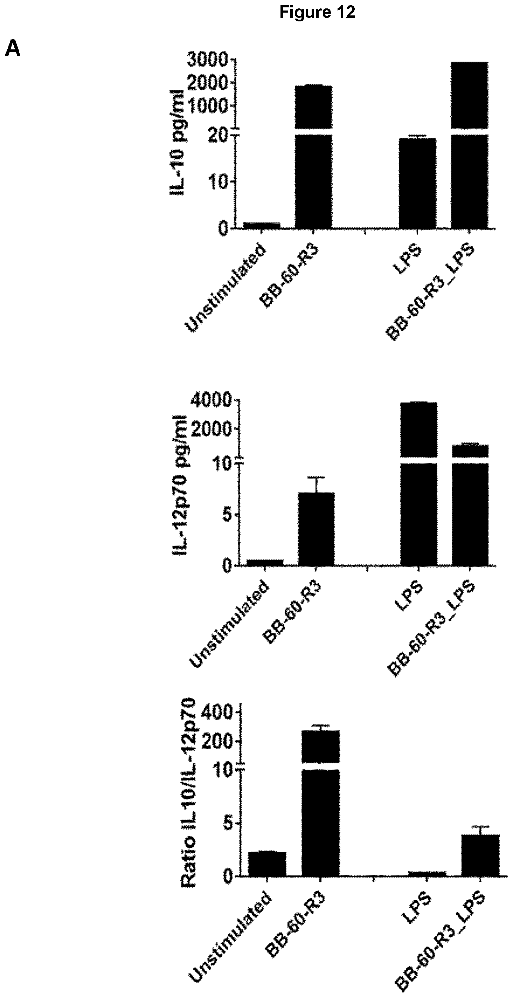

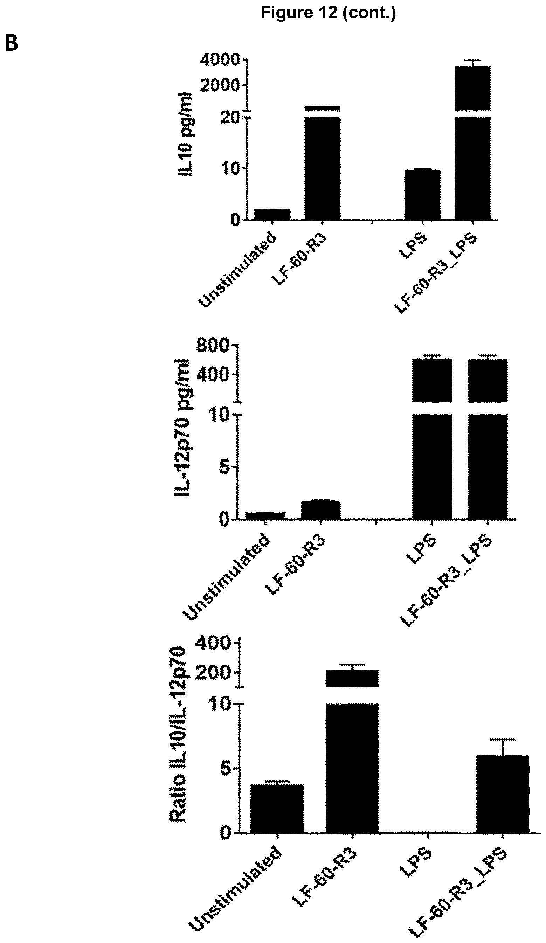

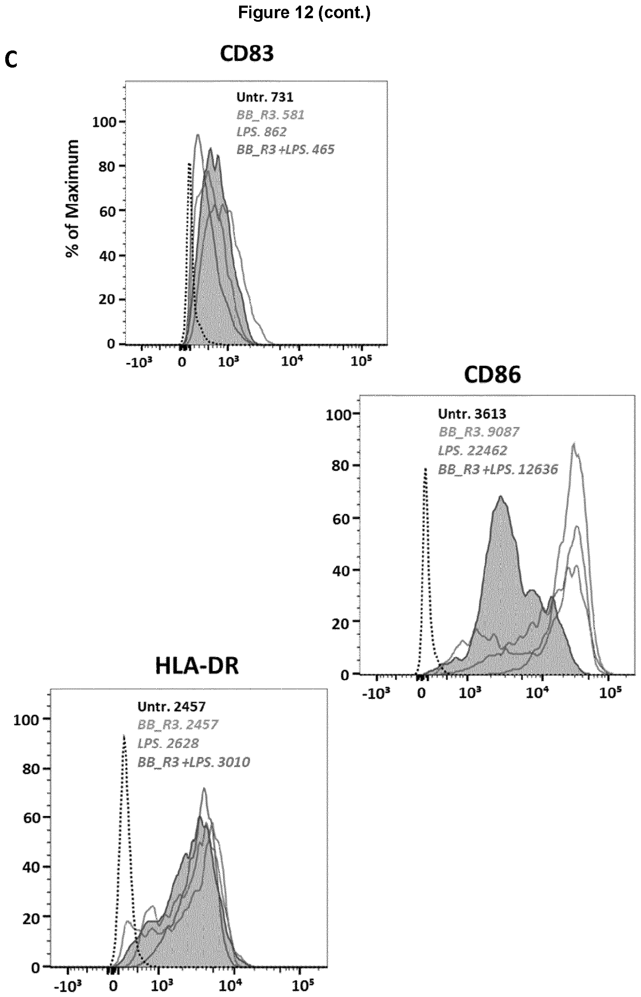

The present invention provides a synthetic composition, such as infant formulae or weaning food, comprising a specific *Bifidobacterium breve* strain (short: B. *breve*), which has been deposited with the DSMZ under the accession number DSM 32583 and is called *inter alia* herein B. *breve* DSM 32583 or DSM 32583 or the like. It was found by the inventors that such particular strain can be applied to any composition as defined herein, which, if applied to a subject, particularly to an infant, prevents said subject from developing specific Interleukin 10 (IL-10) mediated diseases based on the new finding that such strain enhances IL-10 expression in said subject, thereby providing an anti-inflammatory response (see **Figure 8** and **12**). Any disease where IL-10 acts as a mediator so that an increased IL-10 expression triggered by the application of such strain results in catching the development of the disease may be covered by the present invention. The usage of such compositions can be seen as a precautionary measure for said subject which is at risk of developing said IL-10 mediated disease or which is healthy.

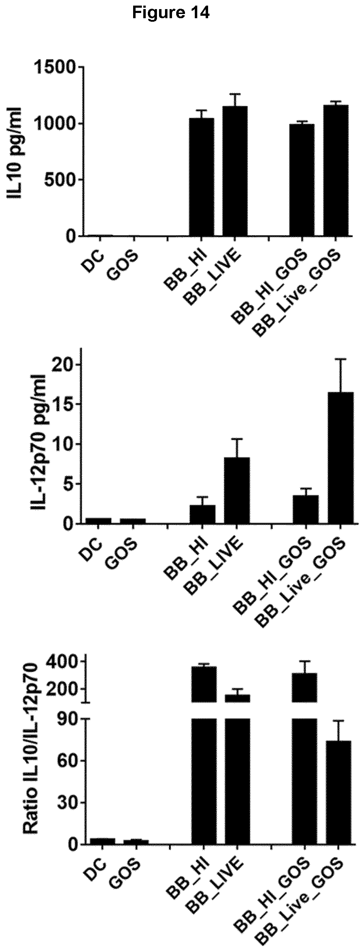

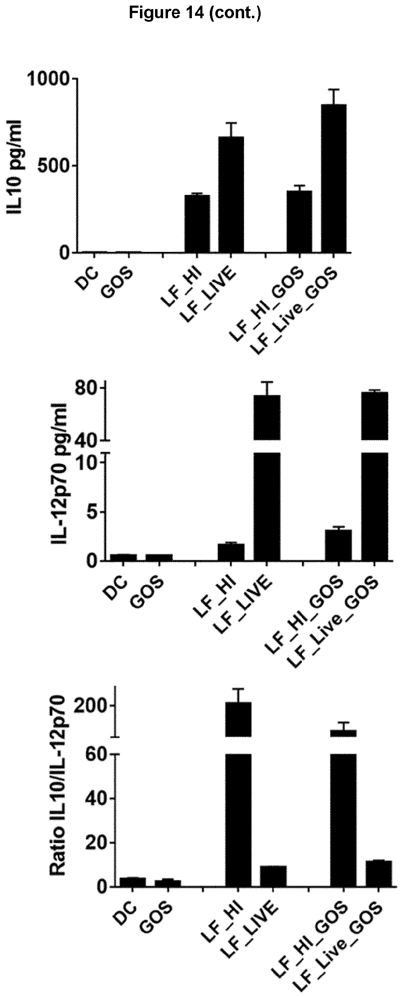

Additionally, the inventors discovered that the abovementioned effect is independent from said strain's physiological state. Meaning, said strain does not need to be in any way stabilized within the composition as defined herein. It can be applied to the composition in preparation in a viable or in a non-viable form as defined elsewhere herein (see **Figure 8** and **14**), e.g. before autoclaving of the composition in a non-viable form. Even the application of a composition comprising the strain in postbiotic form - such cells being mechanically disrupted and/or chemically inactivated (e.g. heat- and/or UV inactivated) - results in the abovementioned effect.

Accordingly, in a first aspect, the present invention relates to a synthetic composition, such as an infant formula, comprising the *Bifidobacterium breve* strain, which is deposited with the DSMZ under the accession number DSM 32583, for use in a method of preventing at least one IL-10 mediated disease by enhancing expression of IL-10 in a subject.

### BRIEF DESCRIPTION OF THE FIGURES

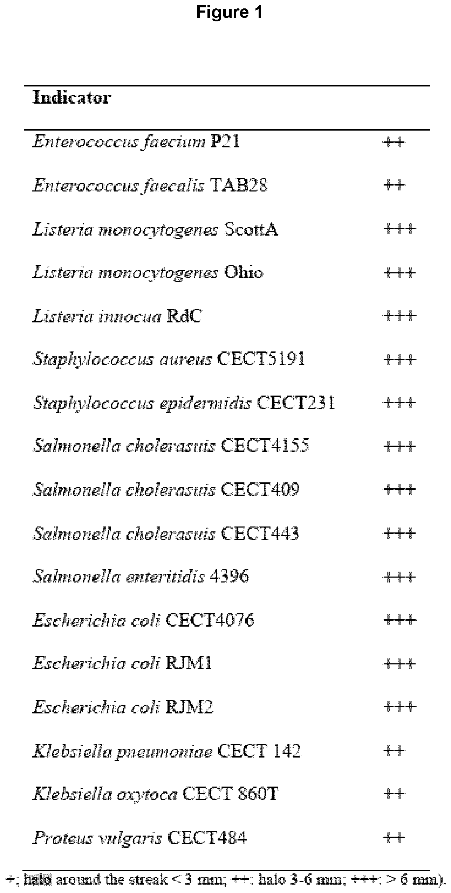

**Figure 1**: Antimicrobial activity of the isolate *B*. *breve* DSM 32583.

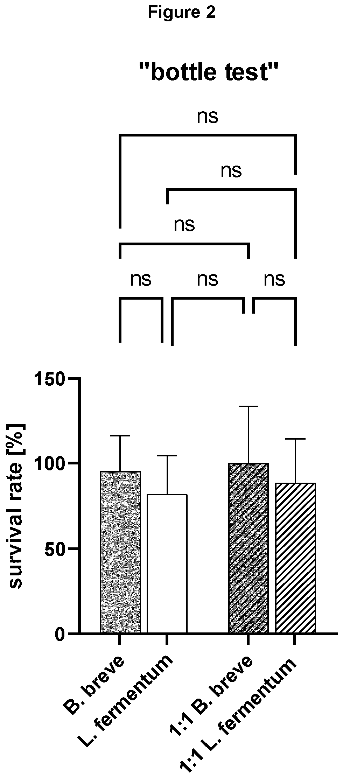

**Figure 2****:** Survival of B. *breve* DSM 32583, *L. fermentum* CECT 5716 or a combination of said two strains after infant's formula preparation ("bottle test": bacteria growth after shaking the formula in the bottle). The survival rate of B. *breve* DSM 32583 is about 93% or more. Non significant (short: ns) comparisons (p>0.05) are depicted by brackets.

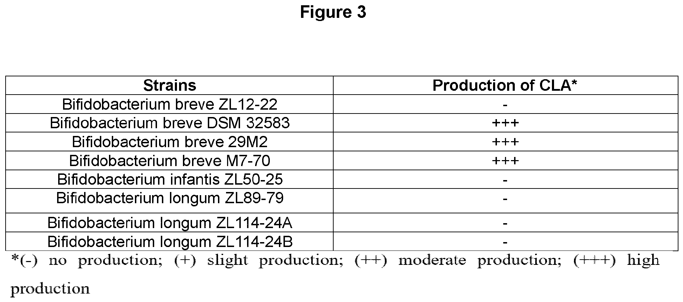

**Figure 3**: CLA production level by the bifidobacterial strains screened.

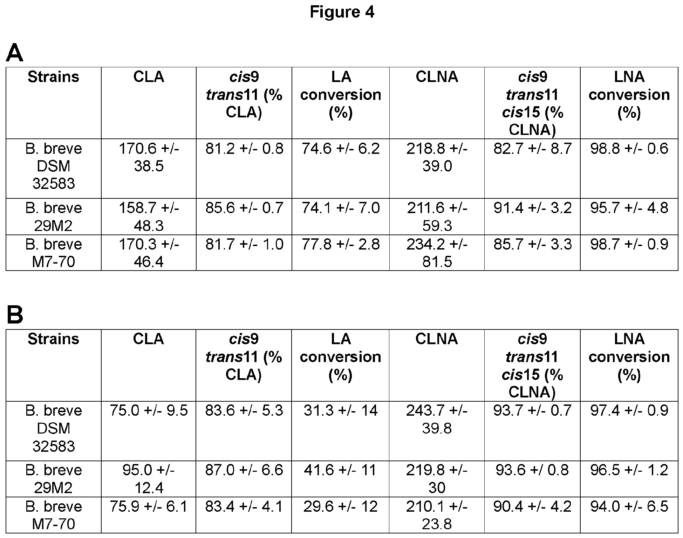

**Figure 4****:** (**A**) CLA and CLNA production (µg/ml) and conversion (%) of LA and LNA by the bifidobacterial strains when growing in MRS-Cys. Values are means of triplicate experiments and standard deviation (± SD). (**B**) CLA and CLNA production (µg/ml) and conversion (%) of LA and LNA by *B*. *breve* strains when growing in skim milk. Values are means of triplicate experiments and standard deviation (± SD).

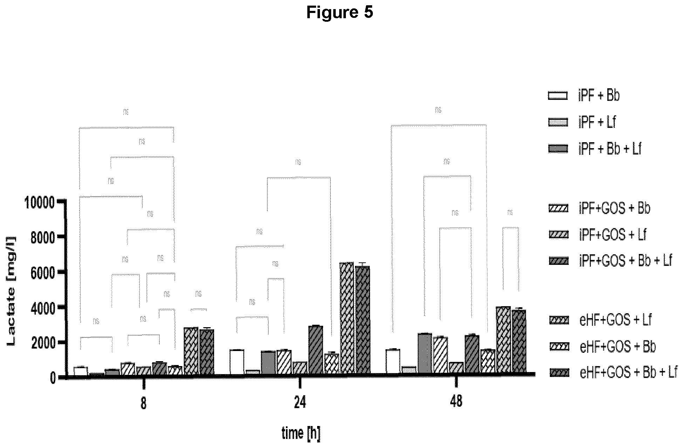

**Figure 5****:** Total lactate production after 8, 24 and 48 h by formula and by (*B. breve* DSM 32583 (short: Bb), *L. fermentum* (short: Lf), or combination (short: Bb + Lf)). Non significant (short: ns) comparisons (p>0.05) are depicted by brackets. No brackets means significant differences between the bars (p<0.05). iPF = intact protein formula; eHF = extensively hydrolysed formula.

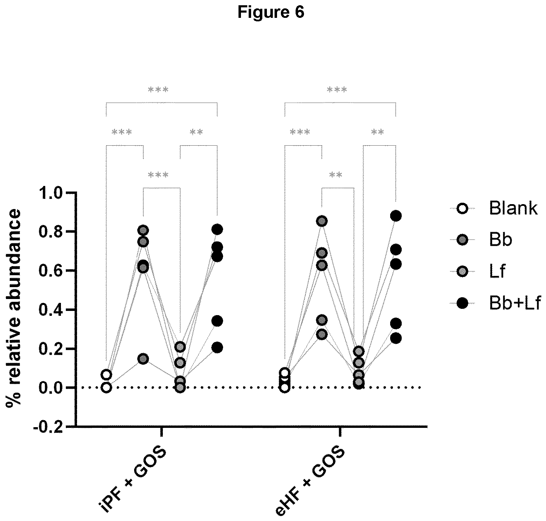

**Figure 6****:** Successful colonization in an ex vivo gut model. The relative abundance (in %) of B. breve DSM 32583 (short: Bb), of L. fermentum CECT 5716 (short: Lf), and of the combination of both strains (short: Bb + Lf) in each composition such as in an intact protein formula (iPF) and in an extensive hydrolyzed formula (eHF) both combined with GOS.

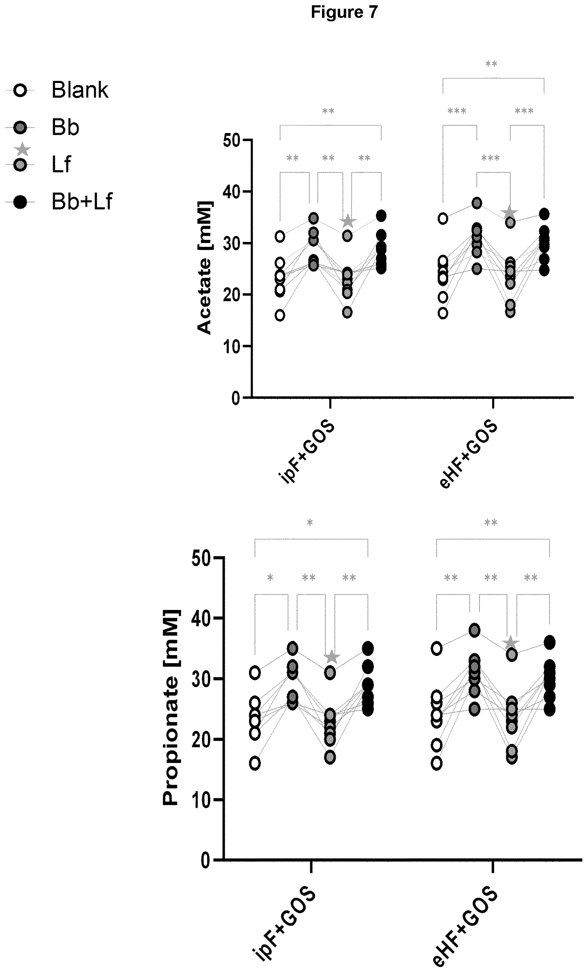

**Figure 7****:** SCFA profile of B. breve DSM 32583 (short: Bb) and/or L. fermentum in different infant formulas.
**Figure 8****:** Effects of *B*. *breve* DSM 32583 and *L. fermentum* CECT 5716 on moDCs key inflammatory cytokines. Bacteria were added to moDCs in different physiolocal states: live, UV-treated at 15W/5×30min, heat inactivated at 60°C/30min or 80°C/3min, R1=1/1, R2=1/10 and R3=1/100 cells to bacteria. R = concentration of the probiotics (relation to the moDCs). (**A**) B. *breve* increases anti-inflammatory IL-10 production in a dose dependent manner - irrespective of viability (both in viable (probiotic) form and heat-inactivated (postbiotic) form). (**B**) *L. fermentum* CECT 5716 increases anti-inflammatory IL-10 production in a dose dependent manner - irrespective of viability (both in viable (probiotic) form and heat-inactivated (postbiotic) form).

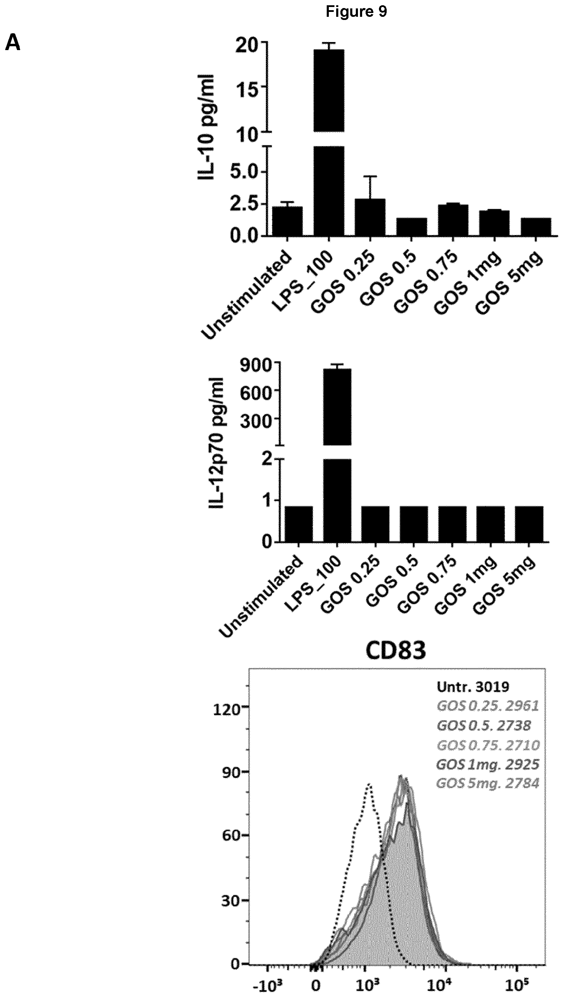

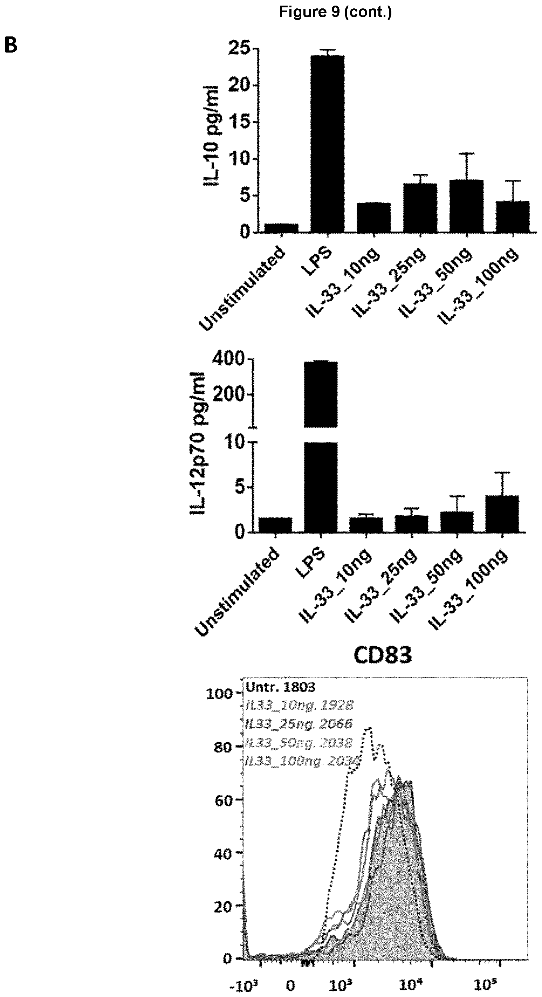

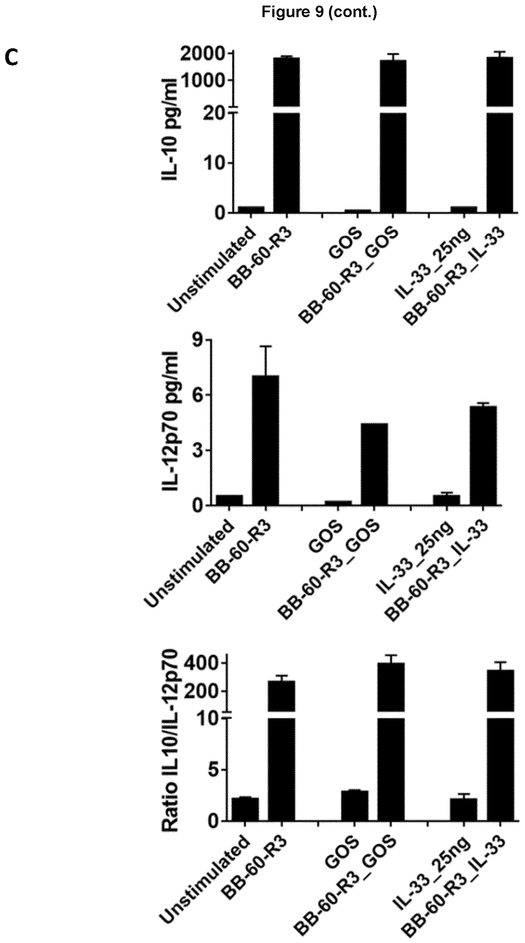

**Figure 9****:** Effects of GOS and IL-33 on moDCs key cytokines and CD83 maturation marker. (**A**) GOS has no effect on moDCs maturation and cytokine production. GOS was added to moDCs at a concentration range of 0, 0.25, 0.5, 0.75, 1 and 5 mg/ml. (**B**) IL-33 per se has no effect on cytokine production by moDCs. IL-33 was added to moDCs at 10, 25, 50 and 100 ng/ml. (**C**) Combination of GOS or IL-33 with heat-inactivated *B*. *breve* DSM 32583 or *L. fermentum* CECT 5716. Heat-inactivated *B*. *breve* induces IL-10 irrespective of GOS and IL-33.

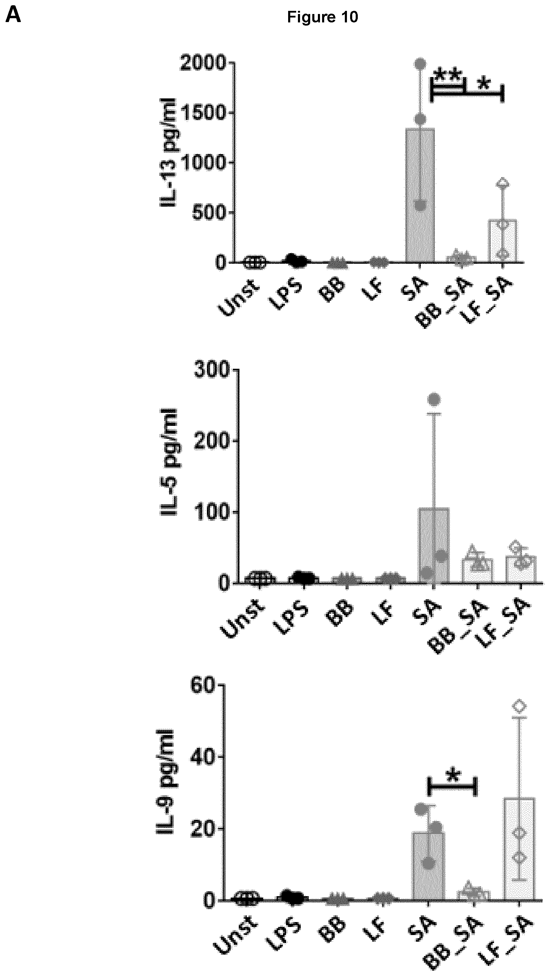

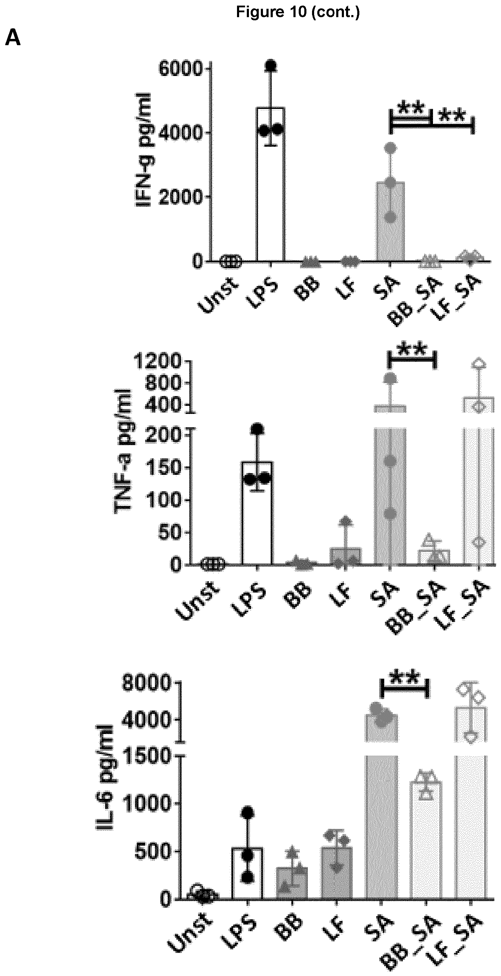

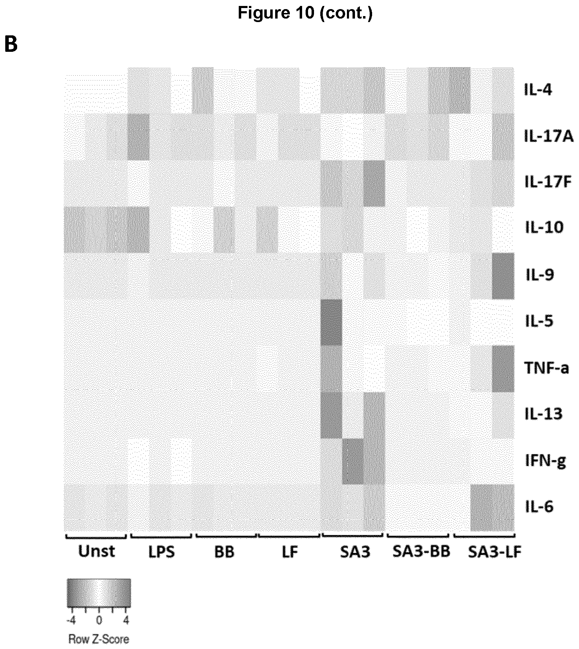

**Figure 10****:** Effects of *B*. *breve* DSM 32583 and *L. fermentum* CECT 5716 on S. *aureus* induced Th2 polarization. Bacteria are added in a heat inactivated state (60°C/30 min) at a ratio of 1/100 cells to bacteria. (**A**) Pro-inflammatory response towards S. *aureus* is reduced by *B*. *breve* and *L. fermentum,* meaning the S. *aureus* induced Th2 response (IL-13, IL-5, IL-9) *in vitro* is dampened. Th1 pro-inflammatory cytokines (IFN-g, TNF-α, IL-6) were also diminished by B. *breve,* while with *L. fermentum* a decrease was only seen with IFN-γ. (**B**) Heatmap shows particular downregulation of Th2 representative cytokines such as IL-4, IL-5, IL-9, and IL-13 as well as of Th1 representative cytokines such as TNF- α, IFN-g, and IL-6 by B. *breve.*

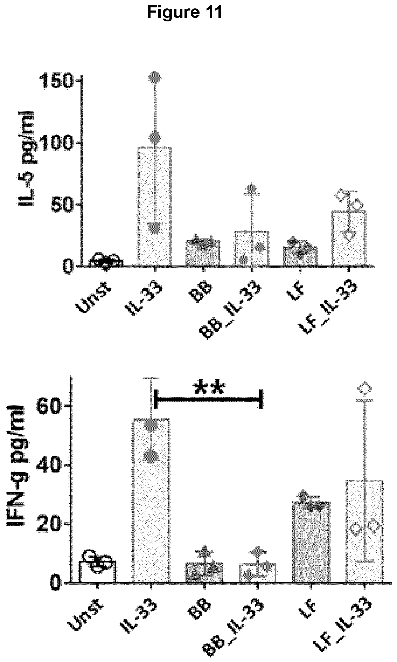

**Figure 11****:** Effects of *B*. *breve* DSM 32583 and *L. fermentum* CECT 5716 on IL-33 induced Th2 polarization. MoDCs were primed with IL-33 (25 ng/ml) or bacteria (60°C/30 min: ratio of 1/100) or their combination, then co-cultured with naive Th cells. IL-33 induced Th2/Th1 response is prevented by heat inactivated *B*. *breve.*
**Figure 12****:** Effects of *B*. *breve* DSM 32583 and *L. fermentum* CECT 5716 on LPS primed moDCs. Bacteria were added in a heat inactivated state (60°C/30 min) at a ratio of 1/100 cells to bacteria. LPS was added at 100 ng/ml. (**A**) A combined action of *B*. *breve* with LPS leads to an upregulation of the anti-inflammatory response in moDCs characterized by a synergistic increase in IL-10 and a clear decrease of IL-12, which is not the case for *L*. *fermentum* (**B**). (**C**) An additional downregulation of CD83, CD86 and HLA-DR maturation markers can also be detected.

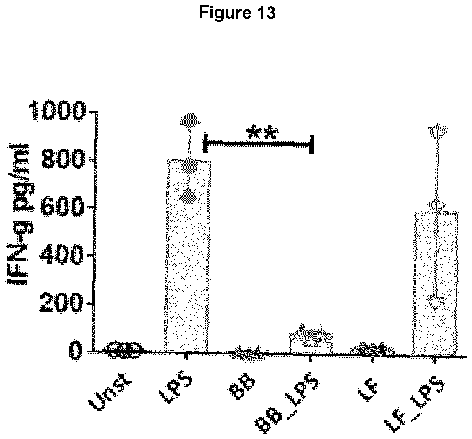

**Figure 13****:** Effects of *B*. *breve* DSM 32583 and *L. fermentum* CECT 5716 on Th cells co-cultured with LPS primed moDCs. MoDCs were primed with LPS (100 ng/ml) or bacteria (60°C/30 min: ratio of 1/100) or their combination, then co-cultured with naive Th cells. B. breve could dampen the Th1 induced inflammation through a significant downregulation of IFN-γ levels.
**Figure 14****:** Effects of live and heat inactivated B. *breve* DSM 32583 and *L. fermentum* CECT 5716 combined or not with GOS on moDCs. Bacteria were added in a live or heat inactivated state (60°C/30 min) at a ratio of 1/100 cells to bacteria, alone or in combination with GOS (0.75 mg/ml). GOS in combination with live *B*. *breve* or *L*. *fermentum* has no additional effect on IL-10 and IL-12 cytokine levels. Thus, GOS has no effect on the anti-inflammatory effect of *B*. *breve* or *L. fermentum* independent of the physiological state.

### DETAILED DESCRIPTION OF THE INVENTION

The inventors provide herein a composition comprising a novel *Bifidobacteroium breve* strain, which was deposited with the DSMZ under the accession number DSM 32583, for use in a method of preventing at least one IL-10 mediated disease by enhancing expression of IL-10 in a subject.

### Strain

Bifidobacteria are gram-positive, nonmotile, often branched, anaerobic bacteria. Bifidobacteria are one of the major genera of bacteria that make up the gastrointestinal tract microbiota in infants. The *B*. *breve* DSM 32583 has been isolated from human breast milk, which makes it particularly suitable and useful for the preparation of infant formulas / formulae (see **Example 2**). The following properties can be attributed to said novel strain:

### Successful colonization of the intestine of human infants in an ex vivo gut model

Early-life colonization of the intestinal tract is a dynamic process influenced by numerous factors. The impact of probiotic-supplemented infant formula on the composition and function of the infant gut microbiota is not well defined. It is however known that exogenous strains provided at early age are often unable to colonize the human intestine. To achieve a physiological effect in the intestine, it is necessary for strains to survive (pass through) the gastrointestinal passage and to settle in the intestine at least temporarily.

The novel B. breve DSM 32583 can colonize the intestine in said ex vivo gut model, as shown in **Figure 6**. DSM 32583 survived the small intestinal passage and remained in the gut as demonstrated with said ex vivo gut model. The supplementation with probiotic *B*. *breve* DSM 32583 increased the amount of Bifidobacteriaceae in most infants (see the appended Examples).

### Genome analysis

The genome of DSM 32583 has been sequenced to confirm its affiliation to Bifidobacterium (see **Example 1**).

### Pathogen defense

A further advantageous technical feature of the novel strain of the present invention, DSM 32583, is its advantageous antimicrobiological effect against different pathogens that are frequently involved in cases of infant infections. Said antimicrobiological effect is characterized by impairing or even inhibiting the growth of a pathogenic bacterium. Said surprising anti microbiological effect (potential pathogen defense) has been examined by the present inventors by applying a well-established overlay assay as it is described in the appended Examples. The results of this assay are depicted in **Figure 1** (see also **Example 3**) which illustrates the remarkable antimicrobiological capabilities of the strain of the invention.

### Survival rate in infant formulas when having contact with oxygen

Anaerobic bacteria have sometimes difficulties to survive in the presence of oxygen (e.g. when preparing infant formulas). The particular strain of the present invention however has a survival rate after bottle preparation for an infant which makes it suitable for the preparation of infant formulas. In theory, the bacterial number added to the bottle before and after preparation should be the same. However, if there is a loss in the viability of the strain, the final concentration will be lower, since some of the anaerobic bacteria die after mixing in the aerobe setting.

The difference between the theoretical and the real concentrations at the end of the preparation is the survival of the strain (see **Figure 2** and **Example 4).** *B. breve* DSM 32583 has a survival rate of at least about 90% in the formula after bottle preparation, which qualifies said strain as a commercial strain for infant formulas.

### Production of riboflavin

B. *breve* DSM 32583 produces riboflavin, as described in **Example 5.**

### Production of CLNA and CLA

B. breve DSM 32583 also produces conjugated linolenic acid (CLNA) from linolenic acid (LNA) (see **Figure 4** and **Example 6**).

In addition, said *B*. *breve* DSM 32583 of the present invention is further capable of producing conjugated linoleic acid (CLA) from linoleic acid (LA) (see **Figure 3** and **Example 6**).

### Total lactate production

Said *B*. *breve* DSM 32583 is additionally capable of producing a total lactate content between about 400 to about 800 mg/L, between about 500 to about 700 mg/L, between about 550 to about 650 mg/L. Lactate was measured in the samples following the lactic acid UV-method kit instructions known to a person skilled in the art. In a preferred embodiment, said strain is capable of producing a total lactate content of about 596 mg/L (see **Table 1** and **Figure 5****, Example 7**).

As can be seen from **Figure 5** and **Table 1** the total lactate content in mg/L increases over time (measured from 8 to 48h) for each composition (see e.g. iPF and iPF+GOS for *B*. *breve* in **Figure 5**) in comparison to the same composition (without galacto-oligosaccharide (short: GOS)). When comparing the compositions to one another, the application of GOS into the compositions of the present invention, preferably into a nutritional composition as defined elsewhere herein, increases the lactate formation, thus *B*. *breve* DSM 32583 is capable of producing a total lactate content between about 600 to about 1000 mg/L, preferably a total lactate content of about 813 mg/L in comparison to the same composition, but not comprising GOS (see **Figure 5** and **Table 1**). Again, as can be seen from **Figure 5** and **Table 1** the total lactate content in mg/L increases over time (measured from 8 to 48h) for each compositions comprising GOS (see e.g. iPF+GOS and eHF+GOS for *B*. *breve* in **Figure 5**).

### Short chain fatty acid profile

The short-chain fatty acids (SCFAs) acetate, butyrate, and propionate (typically occurring in a 3:1:1 ratio) are quantitatively and metabolically the most important microbial end-products of the human colon fermentation process, as they display several physiological effects (Rivière A, Selak M, Lantin D, Leroy F and De Vuyst L (2016) Bifidobacteria and Butyrate-Producing Colon Bacteria: Importance and Strategies for Their Stimulation in the Human Gut. Front. Microbiol. 7:979). Changes in the bacterial ecosystem are associated with changes in the overall metabolic activity. Therefore, changes in metabolites upon probiotic supplementation formula either with intact or hydrolyzed protein was assessed. Supplementation with B. *breve* DSM 32583 or the combination of *B*. *breve* DSM 32583 +Lf (Lactobacillus fermentum CECT5716) yielded significantly higher levels of acetate, as well as propionate, compared to unsupplemented (blank) or Lf-supplemented conditions. SCFA profiles are optimized by B. breve DSM 32583, independent of food or other probiotics; said effect is likewise visible in the ex vivo system (see **Figure 7** and **Example 9**).

### Prevention of IL-10 mediated disease

Surprisingly, as mentioned above it was discovered by the inventors that said novel *B*. *breve* DSM 32583 is additionally capable of preventing at least one IL-10 mediated disease.

In this context and as used throughout herein, the term "IL-10 mediated disease" refers to any disease such as any allergic and/or any inflammatory reaction that is controlled / regulated and suppressed by an enhanced / increased IL-10 expression in a subject, meaning where IL-10 plays an important role as mediator to prevent such disease from developing / manifesting in a subject as defined herein. Any disease where IL-10 shows its effect as a mediator in the body of the subject (e.g. gastrointestinal tract, skin) so that such disease may be prevented / suppressed by the enhanced IL-10 expression triggered by the strain of the present invention as defined elsewhere herein falls under said term.

IL-10 is an important anti-inflammatory cytokine produced by many cell populations. The main biological function of IL-10 is the limitation and termination of inflammatory responses and the regulation of differentiation and proliferation of several immune cells such as T cells, B cells, natural killer cells, antigen-presenting cells, mast cells, and granulocytes (Asadullah et al., 2003, Pharmacological reviews, 55(2), pp. 241-269). IL-10 was initially characterized as a T helper (TH) 2 specific cytokine; however, further investigations revealed that IL-10 production was also associated with T regulatory (Treg) cell responses. The immunosuppressive activity of IL-10 is mediated by the heterodimeric IL-10 receptor (IL-10R1, IL-10R2). Receptor ligation activates JAK/STAT signaling, leading to major changes in the expression profile of immunomodulatory genes, which actually serve to inhibit the release of pro-inflammatory mediators, reduce antigen presentation and phagocytosis, while increasing the inhibitory tolerance and scavenger functions of these cells. In addition, IL-10 can directly or indirectly inhibit inflammation through the release of anti-inflammatory molecules such as interleukin-1 receptor antagonist (IL-1RA), soluble TNFα receptor and interleukin 27 (IL-27) or through physical interactions with T lymphocytes, suppress inflammation, and allergic responses, and enhance regulatory T cell function (lyer and Cheng 2012, Crit Rev Immunol. 32(1): 23-63).

Generally, monocyte-derived dendritic cells (moDCs) as antigen-presenting cells (APCs) present their antigens to naive T helper cells - TH0 lymphocytes - that have been newly released from the thymus and which have not yet been developed into TH2 or TH1 helper cells. The antigen-specific T-cell receptors of TH0 cells recognize the antigenic peptides presented by MHC class II loci on APCs. Cytokines may be exchanged. These composite factors trigger the peptide-specific TH0 cell to differentiate into either a TH2 or a TH1 lymphocyte. Depending on whether TH1 or TH2 cells are formed, different cytokines are released by said cells. The cytokines they produce are known as TH1-type cytokines and TH2-type cytokines.

TH1-type cytokines tend to produce the pro-inflammatory responses responsible for killing intracellular parasites and for perpetuating autoimmune responses. Interferon gamma (IFN-γ) is the main TH1 cytokine. Excessive pro-inflammatory responses can lead to uncontrolled tissue damage (inflammatory reactions), so there needs to be a mechanism to counteract this. The TH2-type cytokines include, but are not limited to, IL-4, IL-5, IL-9 and IL-13 which are associated with the promotion of IgE and eosinophilic responses in atopy, and also IL-10, which has an anti-inflammatory response. In excess, TH2 responses will counteract the TH1 mediated action. The optimal scenario would therefore seem to be that humans should produce a well balanced TH1 and TH2 response, suited to the immune challenge. TH2 cytokines may have crucial functions in the development of allergic hyperreactivity and during initiation and progression of allergic responses. Thus, if there is a TH2 weighted imbalance, meaning an overexpression of the TH2 cytokines, an allergic reaction characterized by an overexpression of at least any one of IL-4, IL-5, IL-9 or IL-13 cytokine might manifest in a subject. Such allergic reaction may be due to an induced hypersensitive TH2 response. If there is a TH1 weighted imbalance, meaning an overexpression of the TH1 cytokines, inflammatory reactions - such as characterized by an overexpression of IFN-γ might manifest in a subject. Such inflammatory reaction may be due to an induced hypersensitive TH1 response (Berger 2000, BMJ 321(7258): 424; Horejs-Höck and Duschl 2003, Bundesgesundheitsblatt - Gesundheitsforschung -Gesundheitsschutz, vol. 46, pp. 211-216; Baraniuk 1997, The Journal of Allergy and Clinical Immunology, vol. 99, issue 22).

The inventors now discovered by applying B. *breve* DSM 32583 that such particular strain is able to enhance / increase the anti-inflammatory IL-10 expression (independent of the physiological state of the strain as define elsewhere herein), which is *inter alia* produced / released by moDCs compared to if no such strain is applied (see **Figure 8** and **14**). Further, such moDCs may present B. *breve* DSM 32583's antigen to TH0 lymphocytes which results in Treg cells being generated from TH0 cells. Treg cells can also produce inhibitory cytokines such as IL-10 and TGF-β and play a role in developing tolerance which keeps the unbalanced TH1 and TH2 response at bay.

Thus, such strain is able to increase / enhance the anti-inflammatory IL-10 response (produced / released from moDCs and/or Treg cells), which suppresses any unwanted disease such as an allergic reaction characterized by an overexpression of at least any one of IL-4, IL-5, IL-9 or IL-13 - triggered by an unbalanced TH2 response as explained above (see **Figure 10****,** **11** and **12**) and/or an inflammatory reaction characterized preferably by an overexpression of IFN-γ - triggered by an unbalanced TH1 response as also explained above (see **Figure 10****,** **11** and **13**), due to the fact that in such disease as defined herein IL-10 plays a crucial role as mediator for suppressing such disease.

In a preferred embodiment, said IL-10 mediated disease comprises an allergic reaction (as defined herein) of the gastrointestinal tract, an allergic reaction of the respiratory tract or an allergic reaction of the skin of said subject. Thus, the present invention may comprise the composition for the use as defined herein, wherein the IL-10 mediated disease is an allergic reaction of the gastrointestinal tract of said subject. Further, the present invention may comprise the composition for the use as defined herein, wherein the IL-10 mediated disease is an allergic reaction of the respiratory tract of said subject. The present invention may also comprise the composition for the use as defined herein, wherein the IL-10 mediated disease is an allergic reaction of the skin of said subject.

An allergic reaction refers to a hypersensitive immune reaction of the body to non-infectious foreign substances in the environment, such as antigens or allergens from viruses / bacteria, plant pollen, food etc. As explained above, allergic reactions in general may be due to a hypersensitive TH2 response so that an imbalanced TH1 and TH2 response exists. Thus, said allergic reaction may be characterized *inter alia* by an overexpression of inflammatory cytokines such as at least any one of IL-4, IL-5, IL-9 or IL-13 (see again **Figures 10** and **11**).

Type-I allergic reactions, namely skin allergy, are thus characterized by a central role for Th2 producing IL-4, IL-5, IL-9, IL-13, IL-31, and the type 2 innate lymphoid cells (ILC2s) expressing the cytokines IL-5, IL-9 and IL-13, both cells orchestrating the so-called type 2 immune response. As it is known to the person skilled, Th2 cells play a key role in an allergic reaction (such as skin allergy) via activation and recruitment of eosinophils, mast cells and basophils in the affected tissues and by mediating the production of allergen-specific IgEs. In this context, the inventors examined the ability of *B*. *breve* DSM 32583 and *L. fermentum* CECT 5716 postbiotic preparations to modulate such Th2 immune response characteristic in type I allergic reactions, namely skin allergy. To induce said Th2 response *in vitro,* the inventors used S. *aureus* which was isolated from skin lesions of patients with atopic dermatitis (AD). The inventors then optimized a Th2 model *in vitro* using human naive T cells co-cultured with said *S*. *aureus* (see **Example 15**) stimulated moDCs. The same was performed for IL-33 stimulated moDCs (see **Example 16**). The cytokine IL-33 is well established as a potent inducer of type 2 immunity mediated by Th2 cells, mast cells, eosinophils, basophils and group 2 innate lymphocytes. The priming of naive T cells with moDCs exposed to either AD patient's derived S. *aureus* isolates or IL-33 induced the release of both Th2 and Th1 cytokines (see **Figures 10** and **11**). Both postbiotics, especially *B*. *breve* DSM 32583, were able to dampen the S. *aureus* and IL-33-induced Th2/Th1 inflammatory response. B. *breve* DSM 32583 exhibited strong immune regulatory effects, through downregulation of Th2 (IL-4, IL-13, IL-5, IL-9) and Th1 (IFN-γ, TNF-α, IL-6) key cytokines (see **Figures 10** and **11**). The increase of the anti-inflammatory IL-10 response as demonstrated e.g. by **Figures 8A** and **12A** combined with the attenuation of *in vitro* particularly induced Th2 immune responses characteristic for skin allergy as demonstrated by **Figures 10** and **11** emphasizes that *B. breve* DSM 32583 displays remarkable immunoregulatory properties and has thus the potential to prevent allergic reaction of the skin.

As known to the person skilled, in humans, IL10 and IL10R play critical roles in controlling immune responses in the intestinal mucosa, supporting the fact that IL-10 can be considered a mediator in the gastrointestinal tract. Preferably, said allergic reaction of the gastrointestinal tract comprises food allergy. The present invention may thus further comprise the composition for the use as defined herein, wherein said allergic reaction of the gastrointestinal tract comprises food allergy. Food allergy is an immune system reaction that occurs soon after eating a certain food. Even a tiny amount of the allergy-causing food can trigger signs and symptoms such as digestive problems (flatulence, constipation, diarrhea), nausea, vomiting, hives, itching and/or swollen airways and the like. In food allergy, the immune response is biased towards a type 2 cytokine-associated phenotype as explained above (Johnston et al. 2014, J Immunol. 192: 2529-2534).

Preferably, said allergic reaction of the respiratory tract comprises asthma and/or rhinitis. The present invention may thus further comprise the composition for the use as defined herein, wherein said allergic reaction of the respiratory tract comprises asthma and/or rhinitis. It has long been evident that allergic asthma is a prototypical type 2-driven disease, which is characterized based on release of type 2 cytokines (such as IL-4, IL-5 and IL-13) by TH2 cells or innate lymphoid cells-type 2 (ILC2), followed by IgE class switching, eosinophil recruitment, and airway hyperresponsiveness (AHR) (Sun et al. 2022, J Asthma Allergy 15:315-326). Studies have shown that plasma IL-10 and IL-13 concentrations were significantly higher in allergic asthmatic patients than normal control subjects (Wong, C.K., et al., 2001. Clinical & Experimental Immunology, 125(2), pp.177-183). Moreover, in animal models of asthma, IL-10 was shown to be capable of inhibiting allergen-induced airway inflammation and non-specific responsiveness (lyer, S.S. and Cheng, G., 2012. Critical Reviews in Immunology, 32(1), pp.23-63), demonstrating IL-10 as mediator also in the respiratory tract. The same TH2 driven cytokine profile can be detected in allergic rhinitis as another allergic reaction of the respiratory tract (Baraniuk 1997, The Journal of Allergy and Clinical Immunology, vol. 99, issue 22).

Further preferably, said allergic reaction of the skin comprises dermatitis such as atopic dermatitis or allergic contact dermatitis. The present invention may thus further comprise the composition for the use as defined herein, wherein said allergic reaction of the skin comprises dermatitis. Dermatitis is a chronic skin disease typically characterized by itchiness, redness and/or a rash. Dermatitis includes atopic dermatitis, allergic contact dermatitis, irritant contact dermatitis, seborrhoeic dermatitis and stasis dermatitis. Therefore, the present invention may comprise the composition for the use as defined herein, wherein said allergic reaction of the skin comprises atopic dermatitis. The present invention may also further comprise the composition for the use as defined herein, wherein said allergic reaction of the skin comprises allergic contact dermatitis. Particularly, atopic dermatitis (AD) is characterized by IgE sensitization to environmental allergens. Acute AD lesions are infiltrated with CD4⁺ TH2 cells, which secrete predominately IL-4, IL-5 and IL-13, supporting a role specifically for altered TH2 responses in the development of AD. Again, it was demonstrated that IL-10 plays an important role as mediator in the TH2 response to antigen and in the development of skin eosinophilia in a murine model of AD (Laouini et al. 2003, J Clin Invest., 112(7): 1058-1066). Further, it has been shown that IL-10 may have a suppressive role in contact and atopic dermatitis (Boyman, O., et al., 2012. Journal of Allergy and Clinical Immunology, 129(1), pp.160-161), demonstrating IL-10 as mediator also in the skin.

In another preferred embodiment, said IL-10 mediated disease comprises an inflammatory reaction. As explained above, an inflammatory reaction in general may be due to a hypersensitive TH1 response so that again an imbalanced TH1 and TH2 response exists. Thus, said inflammatory reaction may be characterized *inter alia* by an overexpression of the pro-inflammatory IFN-γ cytokine (see again **Figure 10****,** **11** and **13**). Thus, an inflammatory reaction is preferably an IFN-γ overexpression-related inflammatory reaction. Even more preferably, an IFN-γ overexpression-related inflammatory reaction comprises inflammatory bowel disease (IBD). Thus, the present invention may further comprise the composition for the use as defined herein, wherein said IFN-γ overexpression-related inflammatory reaction - which can be seen as an IL-10 mediated disease - comprises IBD. Further comprised herein, is the composition for the use as defined herein, wherein said IL-10 mediated disease is IBD.

IBD is an inflammatory disease that can affect any part of the digestive tract such as the colon and the small intestine. Exemplary IBD include Crohn's disease (CD) and ulcerative colitis (UC) as known to the person skilled. An excessive TH1 response has been observed in the inflamed mucosa of IBD patients, such as in CD patients, comprising an increase in the expression of IFN-γ and tumor necrosis factor (TNF)-α. Further comprised herein, is the composition for the use as defined herein, wherein said IL-10 mediated disease comprises CD or UC. In the context of IBD it has been shown that IL-10 may be applied as potent anti-inflammatory agent in the therapy of IBD (Li and He 2004, World J Gastroenterol., 10(5): 620-625), supporting that pro-inflammatory signals in IBD can be counterbalanced by IL-10 produced by *inter alia* Tregs and emphasizing again that IL-10 is a mediator in inflammatory reactions such as IBD.

### Composition

In this context, the term "composition" refers to any kind of composition, which comprises said strain of the present invention, e.g. a nutritional composition, a cosmetic composition or the like. Said composition can be a liquid, a solid, a gel, a paste, an ointment, a capsule, a food product etc. Said cosmetic composition includes, but is not limited to, a toothpaste, a mouthwash, a cream (e.g. for the skin) an ointment, a balm, etc. preferably suitable for an infant (up to 36 months or 3 years as defined herein elsewhere) but it is also suitable for children with an age of 18, 17, 16, 15, 14, 13, 12, 11, 10, 9, 8, 7, 6, 5, or 4 years. Said composition may also be suitable for adults. The term "food product" comprises for example a food bar, a breakfast cereal, a snack food, a cake, a cookie, sweets, ice cream, a fruit composition (apple sauce, Quetschies, etc) and in particular a formula, more preferably an infant formula, but also food product of the dairy industry, such as a yoghurt, a milk, acidified milk, a cheese, etc., preferably suitable for an infant (up to 36 months or 3 years as defined herein elsewhere) but it is also suitable for children with an age of 18, 17, 16, 15, 14, 13, 12, 11, 10, 9, 8, 7, 6, 5, or 4 years. It may also be suitable for adults. The composition comprising said strain may further comprise one or more other ingredients, such as a preservative, an ingestible support, a flavour, a nutritional component such as a (milk)protein, a carbohydrate, a lipid, a fat, GOS, FOS, vitamin etc., which is/are suitable for a food product, in particular for a food product intended and suitable for the ingestion by an infant (up to 36 months or 3 years as defined herein elsewhere), but it is also suitable for children with an age of 18, 17, 16, 15, 14, 13, 12, 11, 10, 9, 8, 7, 6, 5, or 4 years. It may also be suitable for adults. Preferred compositions of the present invention are defined elsewhere herein such as a nutritional composition, a convenience food product, a nutritional supplement, or a care product. Based on the examples mentioned above and elsewhere herein what a composition of the invention may look like (see e.g. [0051] and [0071]), such composition(s) is/are to be understood as "man-made", i.e. synthetic. Meaning, such composition(s) are artificially created rather than naturally occurring. Therefore, the term "synthetic composition" is to be understood as not encompassing human milk. The skilled person may understand the term "human milk" as "human breast milk".

The composition for the use as defined elsewhere may comprise B. *breve* DSM 32583 in a viable form. The term "viable" refers to a living microorganism which is capable of growing under suitable conditions and includes the microorganism in its resting period (e.g. in a non-reconstituted dry infant formula). The term "viable" may also comprise living microorganism as defined above, which is not metabolically active (not capable of producing any metabolites). The viable form of the microorganism as defined herein may also be called "probiotic form".

Said B. *breve* DSM 32583 as defined elsewhere herein, which is comprised by said composition for the use as defined elsewhere and present in a viable form, may be preserved. This is done in order to maintain its viable cells, i.e. the reduction in the viability of said strain is decreased, at least in part. Methods of achieving this are well known. Preservation may include drying, preferably industrial scale drying, such as spray-drying or freeze-drying. Thus, said B. *breve* DSM 32583 as defined elsewhere herein, which is comprised by said composition for the use of as defined elsewhere may be spray-dried and/or freeze-dried, preferably freeze-dried. Also comprised herein, may be the composition for the use as defined elsewhere comprising B. *breve* DSM 32583, wherein said composition contains a food matrix or fluid fermented with said B. *breve* DSM 32583 being preserved such as spray-dried and/or freeze-dried. In this context, B. *breve* DSM 32583 cells may be in a viable form.

The amount of viable cells can be expressed as bacteria counts, which refers to the number of viable bacteria contained per gram dry weight of the composition. The results may be given as bacteria counts/mL for liquids (e.g. when the composition of the present invention refers to a ready-to-drink preterm infant formula), and bacteria counts/g for solids (e.g. any other nutritional composition as described herein). The amount of viable cells is sometimes also expressed as colony forming units (CFU).

The composition for the use as defined elsewhere may alternatively or additionally comprise the strain of the present invention in a "non-viable" form. In this context, the term "non-viable" refers to a microorganism, which is no longer capable of growing under suitable conditions and which is not capable of producing any metabolites. The term "non-viable" comprises partly or wholly mechanically disrupted cells, or otherwise chemically inactivated B. *breve* DSM 32583 cells. The disruption can be done by known methods such as homogenization, osmotic lysis, sonication, and/or pressure cycling (like a French Press). The inactivation can be done by applying a base, acid, heat and/or ultraviolet light (UV) to such viable B. *breve* DSM 32583 cells. The non-viable form of the microorganism as defined herein may also be called "postbiotic form".

The term "partially disrupted cells" refers to cells of the present invention which are only partially broken up for example when mixing said cells with glass spheres (balls) as it is known to a person skilled in the art. These cells may still comprise intact cell membrane segments but also components of said cells are present. The term "wholly disrupted cells" refers to cells of the present invention, which are completely broken up for example by applying ultra-sonication or osmotic lysis.

The composition for the use as defined elsewhere may also comprise B. *breve* DSM 32583 present in a non-viable form, wherein said strain being present in a non-viable form is chemically inactivated and/or mechanically disrupted as defined herein. The present invention may also comprise the composition for the use as defined herein, wherein said B. *breve* DSM 32583 being present in a non-viable form is heat and/or UV-inactivated, and/or mechanically disrupted. Heat inactivation may comprise temperatures from about 60°C to about 80°C which the viable B. *breve* DSM 32583 cells are exposed to to become heat inactivated. UV treatment may comprise UV light at about 15W which the viable B. *breve* DSM 32583 cells are exposed to to become UV inactivated (see **Examples**). As explained elsewhere herein the inventors discovered that such particular strain enhances IL-10 expression in a subject independent of the physiological state of the strain. Meaning, independent whether such strain is applied in a viable or in a non-viable form as defined herein, such as UV inactivated or heat inactivated, an increased IL-10 expression is detected (see **Figure 8**). Especially with regard to the strain in postbiotic form being e.g. heat- and/or UV inactivated, this represents a completely new niche in the production of any compositions then applied to a subject, since such strain can be added to the composition in preparation any time in its non-viable form e.g. before preservation of the composition (such as autoclaving of the composition).

The disruption and/or inactivation as defined herein may be performed on viable cells after having added such viable cells to the composition in preparation. Alternatively or additionally, the disruption and/or inactivation as defined herein may be performed on viable cells before-hand (before the preparation of such compositions) so that the cells being present in a non-viable form as defined herein may be available as a concentrate comprising non-viable cells which may then be added to the compositions in preparation.

The composition for the use as defined elsewhere may alternatively or additionally comprise a food matrix or a fluid fermented with said B. *breve* DSM 32583 as defined herein. The term food matrix or fluid fermented with said B. *breve* DSM 32583 comprises a substrate, wherein said substrate is fermented by said B. *breve* DSM 32583 as defined elsewhere herein. In this context, the term "substrate", which is comprised by the matrix or liquid / fluid to be fermented refers to any protein, fat and/or carbon source known to a person skilled in the art. "Fermented" means that the DSM 32583 of the invention has been grown with/on said substrate for a period of time which is sufficient for the bacteria to convert the substrate into metabolites, either partly or completely. The presence of metabolites may be determined by methods known in the art and may be reflected by a change of the pH in the medium, preferably by an acidification of the medium, e.g., from pH 7 to pH 6.5.

In terms of numerical quantities, said *B*. *breve* DSM 32583 of the present invention or said further bacterium as defined elsewhere herein may be present (preferbaly in viable form) in said compositions as defined elsewhere herein in an amount corresponding to a value within a range between about 10³ bacterial counts or CFU per g dry weight of the composition to about 10¹¹ bacterial counts or CFU per g dry weight of the composition as defined elsewhere herein, such as about 10³, 10⁴, 10⁵, 10⁶, 10⁷, 10⁸, 10⁹, 10¹⁰ or even about 10¹¹. Said *B. breve* DSM 32583 of the present invention or said further probiotic bacterium as defined elsewhere herein may also be present (preferably in viable form) in said compositions as defined elsewhere herein in an amount corresponding to a value within a range between about 10⁴ bacterial counts or CFU/g dry weight to about 10¹⁰ bacterial counts or CFU/g dry weight of the composition, between about 10⁵ bacterial counts or CFU/g dry weight to about 10⁹ bacterial counts or CFU/g dry weight of the composition, between about 10⁶ bacterial counts or CFU/g dry weight to about 10⁸ bacterial counts or CFU/g dry weight of the composition as defined elsewhere herein.

Said composition for the use as defined elsewhere herein is preferably selected from a nutritional composition, a convenience food product, a nutritional supplement, or a care product - all being understood as as "man-made", i.e. synthetic as mentioned above. In a preferred embodiment, said compositions as defined herein are "Bio" or "organic" compositions, which may carry the organic seal being in existence in Germany since 2001, requirements hereto set out in the European Organic Regulation (EU) 2018/848 for example not using any genetic engineering and/or not being irradiated.

A "nutritional composition" means a substance or composition that satisfies at least a portion or all of a subject's nutrient requirements per day. Said nutritional composition is usually to be taken enterally, by a feeding tube directly into the stomach or orally. Preferably, a nutritional composition is for oral use. "Nutritional composition(s)" may refer to liquids, powders, gels, pastes, solids, concentrates, suspensions, or ready-to-use forms of enteral formulas, oral formulas, formulas for infants, for children and/or for adults.

Such nutritional composition of the present invention is preferably a formula as already mentioned above (see in [0051]) and selected from the group consisting of a preterm infant formula, a low birth weight infant formula, an infant formula, a follow-on infant formula, an enteral nutrition formula, and a growing up milk. Preferably, said nutritional composition is in form of a powder or a liquid. Said nutritional composition according to the present invention may comprise intact milk and/or plant protein, or hydrolysed milk and/or plant protein.

The term "formula" means a synthetic nutritional composition intended to serve as a substitute or supplement to breast milk for a preterm or full-term infant. As already defined above, such term may comprise a preterm infant formula, a low birth weight infant formula, an infant formula, a follow-on infant formula, an enteral nutrition formula, or a growing up milk. It is envisaged (as already mentioned below) that the term "formula" does not encompass human milk. Again, in this context the skilled person may understand the term "human milk" as "human breast milk". Thus, a formula is a synthetic nutritional composition not comprising human milk.

The term "preterm infant formula" means an infant formula intended for a preterm infant. In this context, the term "preterm infant" means an infant born prior to 37 weeks gestational age, typically from about 26 to about 34 weeks gestation. The term "low birth weight (LBW) infant" means an infant having a liveborn weight less than 2,500 g at birth, including those less than 1.8 kg at birth. A preterm infant formula of the present invention may refer to a ready-to-drink liquid.

The term "infant feeding formula" or "infant formula", (short: IF) or the like means a foodstuff intended for particular nutritional use by infants during the first months of life, preferably the first four to six months of life and satisfying by itself the nutritional requirements of this category of person (European Commission 2016/127 of 25 September, 2015). It is envisaged that in accordance with Article 2.1 in combination with Annex I point 2. of the European Commission 2016/127 of 25 September, 2015 an infant formula is manufactured from cows' milk or goats' milk proteins, from soya protein isolates alone or in a mixture with cows' milk or goats' milk proteins, or from protein hydrolysates.

"Follow-on infant formula" (short: FOF) means according to the European Commission 2016/127 of 25 September, 2015, a foodstuff intended for particular nutritional use in combination with a diversified diet by infants aged over 6^{th} month onwards. Said foodstuff constitutes the principal liquid element in the progressively diversified diet of this category of person.

The infant formula and the follow-on infant formula can either be in the form of a liquid, ready-to-consumer (ready to use) or concentrated, or in the form of a dry powder that may be reconstituted to form a formula upon addition of water, preferably in the form of a dry powder. Such formulae are well-known in the art. The infant formulae for use herein, including the exemplified formulae described herein, can therefore be prepared by any of a variety of known or otherwise effective formulation or manufacturing methods.

The term "enteral nutrition formula" means a product which nourishes a subject. Said nutrition formula is to be taken enterally via the human gastrointestinal tract or digestive tract, and it usually includes a lipid or fat source and a protein source. Preferably, the enteral nutrition formula is a complete nutrition mix that fulfils all or most of the nutritional needs of a subject.

The term "growing-up milk" (in short: GUM) or "toddler milk" means a milk-based beverage adapted for the specific nutritional needs for infants more than one year old, usually up to 3 years. FOFs and GUMs can be fed to the young children only in combination with increasing amounts of other baby foods as defined elsewhere herein such as pureed fruits, vegetables and other foodstuffs as the process of weaning progresses.

The terms "preterm infant formula", "a low birth weight infant formula", "infant formula", "follow-on infant formula", "enteral nutrition formula", "growing up milk", and the like such as a "human milk fortifier", are to be understood as "man-made", i.e. synthetic nutritional compositions, and do not encompass human milk. Again, the skilled person understands the term "human milk" as "human breast milk". Infant formulae, follow-on infant formulae and growing-up milks which may be aimed at different age groups are known. These infant formulae, follow-on infant formulae and growing up milks aim to meet the requirements of infants and young children at the different ages.

Alternatively, such nutritional composition of the present invention is preferably a "weaning food" which refers to foodstuff for particular nutritional use fulfilling the particular requirements of infants in good health and is intended for use by infants while they are being weaned, or as a supplement to their diet and/or for their progressive adaptation to ordinary food. Such term may comprise "processed cereal-based foods" which shall comply with the compositional criteria specified in Annex I of the Commission Directive 2006/125/EC of 5 December 2006 (see also Article 1.2 a) and Article 5.1 of the abovementioned Commission) and "baby foods other than processed cereal-based foods" which shall comply with the compositional criteria specified in Annex II of the abovementioned Commission (see Article 1.2 b) and Article 5.3 of the abovementioned Commission). A weaning food of the present invention may for example contain fish, meat and/or vegetables.

Said convenience food product as another composition of the present invention is preferably selected from a cereal, a gummy, a cookie or a candy.

Said nutritional supplement as another composition of the present invention is preferably selected from an infant food supplement, a maternal food supplement, a suspension or a human milk fortifier. The nutritional supplements of the present invention may be fed to the infant and/or consumed by the mother of said infant. Nutritional supplements consumed by the mother of said infant may refer to a maternal food supplement as defined herein. Nutritional supplements fed to said infant may refer to an infant food supplement or a human milk fortifier as defined herein. A suspension as defined herein may be fed to said infant and/or consumed by said mother of said infant.

The nutritional supplement can be presented in any form (a liquid such as ready-to-use beverage, or a high energy gel or high energy snack bar served as a tablet, or a powder) and is consumed by said infant / mother of said infant next to consuming regular food. By consuming said maternal food supplement as defined herein the human milk of the mother becomes richer and more nutritious for said infant as defined herein which is then breast fed. In a preferred embodiment, said maternal food supplement is a powder, capsule or suspension.

A suspension, preferably an oil suspension or an aqueous suspension, may also be comprised under the term of nutritional supplement. An oil suspension - such as a vegetable oil suspension - comprising said B. breve DSM 32583, optionally comprising vitamin D or essential further components (e.g. lutein, DHA and/or ARA) or other probiotic bacterial strains may be preferred.

The term "human milk fortifier" means a supplement used to supplement the calories, protein, minerals and vitamins in human milk fed to preterm infants or infants with a low birth weight. Human milk fortification is generally used for all infants who require tube feeding of human milk and for a few infants who require fluid restriction. A human milk fortifier is preferably fed to preterm infants who require additional nutrients to support their growth. Human milk fortifier as defined herein is preferably in the form of a powder or a liquid, most preferably a powder. As already mentioned above, the term "human milk fortifier" is understood as not comprising human milk. Again, the skilled person understands the term "human milk" as "human breast milk".

Said care product as the final composition of the present invention is preferably selected from an oral hygiene care product or a skin care product. The oral hygiene care product preferably refers to a tooth paste. The skin care product preferably refers to a skin cream, lip balm, body care oil or a moisturizing wipe (also called moisturizing tissue) or a diaper insert.

In a further preferred embodiment of the present invention, the composition for the use as defined elsewhere herein comprises a further bacterium, preferably a bacterium which is also suitable to be fed to an infant, such as a bacterium in probiotic or postbiotic form as defined herein. Thus, the composition of the invention may further comprise at least one probiotic bacterium from any one of the family of Bifidobacteriaceae, Carnobacteriaceae, Enterococcaceae, Lactobacillaceae, Streptococcaceae or Leuconostocaceae, preferably from the family of *Lactobacillaceae* or Bifidobacteriaceae, even more preferably from the family of *Lactobacillaceae.* The composition of the invention may further comprise at least one probiotic bacterium from any one of the genus *Carnobacterium, Enterococcus, Limosilactobacillus* and *Lacticaseibacillus* (previously *Lactobacillus*), *Lactococcus, Leuconostoc, Oenococcus, Pediococcus, Streptococcus, Tetragenococcus, Vagococcus* and *Weissella,* the genus Limosilactobacillus being preferred. More preferably, the at least one probiotic bacterium additionally comprised by the composition as defined herein is *Limosilactobacillus fermentum,* and/or *Lacticaseibacillus rhamnosus,* or any mixture thereof even more preferably, *Limosilactobacillus fermentum,* most preferably the further probiotic bacterial strain is *Limosilactobacillus fermentum* which is deposited with the CECT under the accession number CECT5716 (short. *L. fermentum* CECT 5716 or CECT 5716 strain or the like). In case a further Bifidobacterium species is additionally present in the defined composition of the present invention, it is preferably selected from the group consisting of B. *infants, B. lactis, B. bifidum, B. catenulatum, B. adolescentis, B. thermophilum, B. gallicum, B. animalis, B. angulatum, B. pseudocatenulatum, B. thermacidophilum* and B. *longum* or any mixture thereof.

The composition according to the invention may contain from 10³ to 10¹¹, such as about 10³, 10⁴, 10⁵, 10⁶, 10⁷, 10⁸, 10⁹, 10¹⁰ or even about 10¹¹ bacterial counts or CFU per g of composition on a dry weight basis of said further probiotic bacterial strain as defined elsewhere herein (preferably in viable form). Preferably, *L. fermentum* CECT 5716 as further probiotic bacterial strain as defined elsewhere herein may also be present in said compositions as defined above in an amount corresponding to a value within a range between about 10⁴ bacterial counts or CFU/g dry weight to about 10¹⁰ bacterial counts or CFU/g dry weight of the composition, between about 10⁵ bacterial counts or CFU/g dry weight to about 10⁹ bacterial counts or CFU/g dry weight of the composition, between about 10⁶ bacterial counts or CFU/g dry weight to about 10⁸ bacterial counts or CFU/g dry weight of the composition as defined elsewhere herein.

The abovementioned definitions as disclosed for *B*. *breve* DSM 32583 may also be applicable to said further probiotic bacterium, particularly to *L. fermentum* CECT 5716 which may also be comprised within the composition of the invention comprising said B. *breve* DSM 32583.

In a preferred embodiment, the composition for the use as defined herein, such as a nutritional composition, a convenience food product, a nutritional supplement, or a care product, comprises any one of a non-digestible prebiotic oligosaccharide, a long-chain polyunsaturated fatty acid (short: LC-PUFAs), a vitamin, a mineral, a protein, a fat, or a combination thereof. In the context of the present invention, the term "prebiotic" preferably refers to one or more non-digestible oligosaccharides. Advantageously, the non-digestible oligosaccharide is water-soluble.

More specifically, the non-digestible prebiotic oligosaccharide being comprised by the present composition as defined herein may comprise any one of fructo-oligosaccharide, non-digestible dextrin, galacto-oligosaccharide, xylo-oligosaccharide, arabino-oligosaccharide, arabinogalactooligosaccharide, gluco-oligosaccharide, glucomannooligosaccharide, galactomanno-oligosaccharide, mannanoligosaccharide, chito-oligosaccharide, uronic acid oligosaccharide, sialyl-oligosaccharide, fuco-oligosaccharide, bovine milk oligosaccharide (BMOs), or human milk oligosaccharide (HMOs), which are structurally identical to the oligosaccharides in actual human milk and are produced during the whole lactation period. In a preferred embodiment the non-digestible prebiotic oligosaccharide comprises HMOs.

The present non-digestible oligosaccharide comprised in said compositions as defined herein is most preferably galacto-oligosaccharides (short: GOS), in particular β-galacto-oligosaccharides. In a particular preferred embodiment the present composition comprises between about 0.2 g/100 ml to about 0.8 g/100 ml GOS.

Additionally or alternatively to non-digestible prebiotic oligosaccharides, the composition may comprise long-chain polyunsaturated fatty acids. LC-PUFAs may comprise docosahexaenoic acid (short: DHA), a linoleic acid (short: omega-6), linolenic acid (short: omega-3), eicosapentaenoic acid (short: EPA), arachidonic acid (short: ARA), or any combination thereof. The LC-PUFA may be provided as free fatty acids, in triglyceride form, in diglyceride form, in monoglyceride form, in phospholipid form, or as a mixture of one of more of the above. Preferably, the composition comprises a long-chain polyunsaturated fatty acid which is any one of DHA, ARA, or a combination thereof. The amount of LC-PUFA in the composition of the invention (preferably any one of the nutritional composition as defined in [0063] above selected from the group consisting of a preterm infant formula, a low birth weight infant formula, an infant formula, a follow-on infant formula, an enteral nutrition formula, and a growing up milk) may be at least about 5 mg/100 kcal. The composition (preferably any one of the nutritional composition as defined in [0063] above) may comprise LC-PUFA in an amount from about 5 mg/100 kcal to about 140 mg/100 kcal. Preferably, the composition (preferably any one of the nutritional composition as defined in [0063] above) may comprise LC-PUFA in an amount from about 10 mg/100 kcal to about 70 mg/100 kcal, most preferably between about 15 mg/100 kcal to about 47 mg/100 kcal.

The composition of the invention (preferably any one of the nutritional composition as defined in [0063] above) may comprise about 5 mg/100 kcal to about 100 mg/100 kcal of DHA. Preferably, the composition (preferably any one of the nutritional composition as defined in [0063] above) may comprise about 10 mg/100 kcal to about 50 mg/100 kcal of DHA. The composition (preferably any one of the nutritional composition as defined in [0063] above) may comprise about 5 mg/100 kcal to about 140 mg/100 kcal of ARA. Preferably, the composition (preferably any one of the nutritional composition as defined in [0063] above) may comprise about 10 mg/100 kcal to about 70 mg/100 kcal of ARA. The composition may comprise both DHA and ARA. The weight ratio of ARA:DHA may be between about 1.5:1. The composition may comprise oils containing DHA and/or ARA. The DHA and ARA may be in a natural form or in a refined form.

Additionally or alternatively, the composition of the present invention may contain all vitamins and minerals understood to be essential in the daily diet and in nutritionally significant amounts. Minerals are usually added in salt form. The infant formula may optionally contain other substances which may have a beneficial effect such as folate, choline, nucleotides, nucleosides, and the like.

Additionally or alternatively, the proteins that may be utilized in the nutritional composition, in the convenience product or in the nutritional supplement or even in the care product include any proteins or nitrogen source suitable for human consumption. Such proteins are well known by those skilled in the art and can be readily selected when preparing such products. The composition according to the present invention (preferably any one of the nutritional composition as defined in [0063] above) may contain a protein source in an amount of between about 1.5 to about 3 g/100 kcal, preferably between about 1.6 to about 2.8 g/100 kcal. Also preferred as a protein additionally comprised by the compositions as defined herein is any one of a milk protein concentrate containing milk fat globule membrane (MFGM), a MFGM protein, a hydrolysate, a collagen peptide or a cytokine. For the present invention a protein may also include peptides and free amino acids. The proteins may be intact or hydrolysed or a mixture of intact and hydrolysed proteins.

Additionally or alternatively, examples of suitable fat sources / fats typically incorporated into said compositions include high oleic safflower, sunflower oil, soy oil, fractionated coconut oil (medium chain triglycerides, MCT oil), corn oil, canola oil, coconut, palm and palm kernel oils, marine oil, cottonseed oil, bovine or goat milk fat and specific fatty acids as defined elsewhere herein such as DHA and ARA. The composition according to the present invention (preferably any one of the nutritional composition as defined in [0063] above) may contain a fat source in an amount of between about 3 to about 6 g/100 kcal, preferably between about 3.5 to about 5.5 g/100 kcal.

The carbohydrates that may also be used in said compositions can vary widely. Examples of carbohydrates suitable for (preterm) infants typically include hydrolyzed corn starch, maltodextrin, saccharose, glucose polymers, sucrose, corn syrup, corn syrup solids, rice syrup, glucose, fructose, lactose, high fructose corn syrup and non-digestible prebiotic oligosaccharides such as GOS as defined elsewhere herein. Any single carbohydrate listed above, or any combination thereof, as appropriate may be utilized. Preferably, the carbohydrate additionally comprised by the compositions defined herein besides GOS is lactose. The composition according to the present invention (preferably any one of the nutritional composition as defined in [0063] above) may contain a carbohydrate source in an amount of between about 5 to about 15 g/100 kcal, preferably between about 7 to about 11 g/100 kcal.

In a most preferred embodiment, the compositions, in particular any nutritional composition as defined in [0063] above, comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- between about 5 to about 22 wt% (w/w)% of a protein source, preferably between about 9 to 13 wt% of a protein source based on dry weight of the composition;
- between about 40 to about 66 wt% of a carbohydrate source, preferably between about 47 to 56 wt% of a carbohydrate source based on dry weight of the composition;
- between about 1 to about 12 wt% of a non-digestible oligosaccharide (in particular GOS), preferably between about 2 to 6 wt% of a non-digestible oligosaccharide (in particular GOS) based on dry weight of the composition;
- between about 20 to about 34 wt% of a fat source, preferably between about 24 to 28 wt% of a fat source based on dry weight of the composition;
- between about 0.057 to about 0.106 wt% of an ARA source, preferably between about 0.059 to 0.104 wt% of an ARA source based on dry weight of the composition; or
- between about 0.041 to about 0.164 wt% of a DHA source, preferably between about 0.059 to 0.114 wt% of a DHA source based on dry weight of the composition.

In an alternative most preferred embodiment, the compositions, in particular any nutritional composition as defined by [0063] above, comprises at least any one of:
- a total energy content of between about 450 to about 540 kcal/100 g, preferably between about 470 to about 520 kcal/100 g, most preferably between about 505 to about 515 kcal/100 g based on dry weight of the composition;
- a protein source in an amount of between about 1.5 to about 3 g/100 kcal, preferably between about 1.6 to about 2.8 g/100 kcal;
- a carbohydrate source in an amount of between about 5 to about 15 g/100 kcal, preferably between about 7 to about 11 g/100 kcal;
- a fat source in an amount of between about 3 to about 6 g/100 kcal, preferably between about 3.5 to about 5.5 g/100 kcal;
- a DHA source in an amount of between about 5 mg/100 kcal to about 100 mg/100 kcal, preferably, between about 10 mg/100 kcal to about 50 mg/100 kcal of DHA; or
- an ARA source in an amount of between about 5 mg/100 kcal to about 140 mg/100 kcal, preferably between about 10 mg/100 kcal to about 70 mg/100 kcal.

In a particular embodiment, if the nutritional composition is a preterm infant formula, which is a ready-to-drink liquid, said preterm infant formula, as defined elsewhere herein comprises at least any one of:
- a total energy content of between about 70 to about 86 kcal/100 ml, preferably between about 76 to about 80 kcal/100 ml;
- between about 2 to about 6 g/100 kcal of a protein source, preferably between about 3.2 to 3.65 g/100 kcal of a protein source;
- between about 9 to about 12 g/100 kcal of a carbohydrate source, preferably about 10.5 g/100 kcal of a carbohydrate source;
- between about 4 to about 6 g/100 kcal of a fat source, preferably between about 4.85 to 5.1 g/100 kcal of a fat source;
- between about 30 to about 40 mg/100 kcal of an DHA source, preferably between about 33 to 36 mg/100 kcal of an DHA source; or
- between about 44 to about 63 mg/100 kcal of a ARA source, preferably between about 50 to 55 mg/100 kcal of a ARA source.

The composition for the use as defined herein is intended for any subject, preferably a mammal, even more preferably a human. The subject may be any age. The subject may be an infant, a child, or even an adult, preferably an infant. The composition for the use as defined herein is preferably intended for an infant, optionally wherein said infant is a preterm infant. The term "infant" includes a child with an age of 3 years or 36 months or below. The term "infant" as used herein, includes a toddler at that age, actual or corrected, including individuals from 0 to 36 months of age, actual or corrected. The term "infant" comprises a "preterm infant" as defined herein or a "full-term infant". According to the present invention, the infant may be a preterm infant. In other words, said composition may be suitable for an infant (preterm or full-term infant) according to the present invention. Preferably, said infant to which the composition is to be fed to / is suitable for, is an infant (preterm and/or full-term infant) receiving human milk. Also preferably, said infant to which the composition is to fed to / is suitable for, is a preterm infant or a preterm infant receiving human milk.

A preterm infant formula is intended for preterm infant being born prior to 37 weeks gestational age. An infant formula is intended for a full-term infant during the first months of life, preferably the first 4 to 6 months of life. A follow-on formula is intended for a full-term infant over the age of 6 months. A growing-up milk is intended for a full-term infant (toddler) with an age of 12 months or above, usually up to 3 years.

A weaning food is intended for a full-term infant during the first years of life, particularly an infant (toddler) up to 36 months of age. An infant formula, a follow-on formula, a growing-up milk or a weaning food as defined herein may also be suitable for a preterm infant as soon as the preterm infant has compensated its deficiency of being born too early and has reached a developmental stage similar to a full-term born infant.

The subject being addressed in the invention and as defined herein is preferably one that is at risk of developing an IL-10 mediated disease. The present invention may also comprise the composition for the use as defined herein, wherein the subject as defined herein is at risk of developing an IL-10 mediated disease. Being at risk of developing such disease means that the subject as defined herein does not yet suffer from said disease, but is susceptible to the development of such disease. If the composition as defined herein is applied to said subject, said subject will not develop such disease. In one embodiment of the invention, the subject is at risk of developing an allergic reaction of the gastrointestinal tract as defined herein. Preferably, the subject is at risk of developing food allergy as defined herein. In another embodiment of the invention, the subject is at risk of developing an allergic reaction of the respiratory tract as defined herein. Preferably, the subject is at risk of developing asthma and/or rhinitis as defined herein. In a further embodiment of the invention, the subject is at risk of developing an allergic reaction of the skin as defined herein. Preferably, the subject is at risk of developing dermatitis such as AD as defined herein. In a further embodiment of the invention, the subject is at risk of developing inflammatory bowl disease as defined herein. In a further embodiment of the invention, the subject is at risk of developing CD or UC as defined herein.

Alternatively, the subject being addressed in the invention and as defined herein is preferably one that is healthy. The present invention may also comprise the composition for the use as defined herein, wherein the subject as defined herein is healthy. Meaning, such subject does not suffer from said disease as mentioned herein but is also not susceptible to the development of such disease. However, if the composition as defined herein is applied to said subject, said subject will remain healthy or will become even healthier due to the immune boosting effect of the composition.

Said composition may particularly be suitable for enteral feeding to said infant. The composition is enterally fed, and the term "enteral" is intended to refer to the delivery directly into the gastrointestinal tract of the infant. Most preferably, said composition according to the present invention is fed to said infant orally.

In a further preferred embodiment, said infant as defined herein to which the composition is suitable for, is delivered by cesarean section (hereinafter referred to as 'a C-section infant'). Alternatively, the infant may have been vaginally delivered. To limit the negative impact associated with the delivery by caesarean section on the development of an early bifidogenic intestinal microbiota, it is preferred that the infant formula of the present invention is fed at least within the first hours after delivery.

The composition may be in any form known in the art to be suitable for such feeding, such as in solid form, in semi-solid form or in liquid form. Preferably, the composition as defined elsewhere herein is in the form of a reconstitutable powder, a tablet, a flake, a softgel, a creme, a suspension or a ready-to-use liquid. A "reconstitutable powder" is a powder which is reconstituted with a suitable aqueous fluid, typically water or milk, to obtain / form a liquid composition for immediate oral or enteral use. Such powder includes spray dried, agglomerated, dry mixed or other known or otherwise effective particulate form. The quantity of such powder required to produce a volume suitable for one serving can vary. Preferably, the present composition, when in the form of a powder, a tablet, a flake, a creme, a softgel, a suspension or a ready-to-use liquid is contained within a container, preferably a stick or stickpack or a sachet. A "ready-to-use" liquid as used herein refers to a liquid suitable for feeding an infant right away, which is easy to drink or eat, and wherein no other components or additional water is/are added. A ready-to-use liquid may comprise a ready-to-feed liquid or ready-to-drink liquid (such as a preterm infant formula).

The nutritional composition of the present invention as defined elsewhere may also be suitable as the sole nutrition source for said infant, in particular for said preterm infant as defined elsewhere herein. Since the nutritional compositions according to the present invention can be used as a sole source of nutrition, it comprises a protein source, a lipid source, a carbohydrate source, vitamins, and minerals in amounts sufficient to maintain an infant's health (i.e., to prevent malnutrition), and optionally comprise folate, choline, nucleotides, nucleosides, GOS, HMOs and the like.

It is noted that as used herein, the singular forms "a", "an", and "the", include plural references unless the context clearly indicates otherwise. Unless otherwise indicated, the term "at least" preceding a series of elements is to be understood to refer, if not particularly defined differently, to one or more such as two, three, four, five, or more. The term "and/or" wherever used herein includes the meaning of "and", "or" and "all or any other combination of the elements connected by said term".

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified. The term "including" means "including but not limited to". "including" and "including but not limited to" are used interchangeably.

The term "about" means plus or minus 10%, preferably plus or minus 5%, more preferably plus or minus 2%, most preferably plus or minus 1%.

Throughout the description and claims of this specification, the singular encompasses the plural unless the context otherwise requires and *vice versa.* In particular, where the indefinite article is used, the specification is to be understood as contemplating plurality as well as singularity, unless the context requires otherwise.

A better understanding of the present invention and of its advantages will be gained from the following examples, offered for illustrative purposes only. The examples are not intended to limit the scope of the present invention in any way.

### EXAMPLES OF THE INVENTION

### Material and Methods

### Example 1: Whole genome sequencing.

*B. breve* DSMZ 32583 was deposited as DSM 32583 in the German Collection of Microorganisms.

DNA was extracted using CTAB (Murray & Thompson, 1980) and fragmented (Covaris sonicator model E220, Covaris, UK). Libraries were prepared using the TruSeq DNA kit. Sequencing was conducted on a MiSeq using a PE300 v3 cartridge, generating 8,943,755 raw reads. Reads were checked using fastqc v0.72 and adapter were removed using Clip v1.0.3. The genome was assembled using Unicycler v0.4.6.0 with default settings and annotated with PROKKA v1.13.3 both implemented in Galaxy.

The genome of DSMZ 32583 comprises 2,292,381 bp in 11 contigs (*N₅₀* = 657,788 bp), with a G+C content of 58.74%, 1,951 coding sequences (CDS), 2 rRNAs and 54 tRNAs. It shared 98.2% average nucleotide identity with the genome of type strain *B*. *breve* DSM 20213^{T} (GCA_001025175.1), confirming its affiliation to *Bifidobacterium.* Genome completeness was estimated to be 100% at the taxonomic level of family using CheckM v1.0.18.. No bacteriocin or similar genes were detected through BAGEL4 and BACTIBASE.

### Example 2: Isolation from human milk and identification.

*Bifidobacterium breve* DSM 32583 was isolated in the frame of a study to evaluate the lactobacilli and bifidobacterial diversity of human milk. Women were enrolled in this study according with the following criteria: (a) healthy women without present or past underlying conditions (including mastitis); (b) normal full-term pregnancy; and (c) absence of infant and/or maternal perinatal problems (including mastitis). Milk samples were collected in sterile tubes by manual expression using sterile gloves. Previously, nipples and surrounding skin were cleaned with soap and sterile water, and soaked in chlorhexidine. The first drops (~1 ml) were discarded. The milk and skin samples were obtained at day 7 after delivery and kept at 4°C until delivery to the laboratory, which happened within the first three hours after collection. The study from which this strain was isolated was was approved by the Ethical Committee on Clinical Research of Hospital Clinico (Madrid).

Peptone water dilutions of the milk samples were plated in triplicate onto de Man, Rogosa, and Sharpe (MRS, Oxoid, Basingstoke, UK) supplemented with L-cysteine (0.5 g/L) (MRS-Cys) and TOS agar plates, which were incubated anaerobically (85% nitrogen, 10% hydrogen, 5% carbon dioxide) in an anaerobic workstation (MINI-MACS, DW Scientific, Shipley, UK) at 37°C for 48 h. In this study, presence of bifidobacteria in human milk was investigated. Colonies were obtained from all the milk samples in MRS-Cys plates. The MRS-Cys and TOS counts ranged from 1.45 × 10² to 1.25 × 10⁴ CFU/ml. Such bacterial concentration values are similar to that reported from hygienically-obtained human milk. No growth was detected on VRBA plates, which confirmed the hygienic collection of the milk samples. A total of 104 colonies were selected from MRS-Cys or TOS plates for further characterization. Subsequently, they were inoculated in MRS broth tubes, which were incubated aerobically to exclude those with fastidious incubation requirements (<10⁸ CFU/ml after 24 h at 37°C). The isolates were examined by phase-contrast microscopy to determine cell morphology and Gram-staining reaction, and tested for oxidase and catalase activities. All the Gram-positive and catalase-negative isolates with a typical bifidobacterial shape were identified to the genus level (*Bifidobacterium*) by demonstration of fructose-6-phosphate phosphoketolase (F6PPK) activity in cellular extracts.

### Example 3: Antimicrobial activity.

An overlay method previously described (Magnusson & Schnürer, 2001; Martin et al. 2005b) was used to determine the ability of the strain to inhibit the growth of different bacteria. After the incubation, zones of inhibition around the strain streaks were examined and halo's diameters were measured. The following bacteria were employed as indicator organisms: *Enterococcus faecium* P21, *Enterococcus faecalis* TAB28, *Listeria monocytogenes* ScottA, *List. monocytogenes* Ohio, *Listeria innocua* RdC, *Staphylococcus aureus* CECT 5191, *Staphylococcus epidermidis* CECT 231, *Salmonella cholerasuis* CECT 4155, *Sal. Cholerasuis* CECT 409, *Sal. cholerasuis* CECT 443, *Salmonella enteritidis* 4396, *Escherichia coli* CECT 4076 (O157:H7), *E. coli* RJM1, *E. coli* RJM2, *Klebsiella pneumoniae* CECT 142, *Klebsiella oxytoca* CECT 860T and *Proteus vulgaris* CECT 484 (see Figure 1). The plates overlaid with bacterial indicators were incubated at 37°C for 48 h, while those overlaid with yeasts cells or fungal spores were incubated at 30°C for up to 120 h. The plates were examined for zones of inhibition around the strain streaks. All experiments assaying inhibitory activity were performed in triplicate.

In parallel, the strain was grown in MRS broth at 37°C until early stationary phase (A620 ~1.0). Preparation of cell-free supernatants and analysis of their bacteriocinogenic activity were performed as described (Martin et al., 2005b). The microorganisms employed as indicators of bacteriocinogenic activity were the Gram-positive bacteria previously used for determination of the antimicrobial spectrum: *E. faecium* P21, *E. faecalis* TAB28, *P*. *acidilactici* 347, *L*. *monocytogenes* ScottA, *L. monocytogenes* Ohio, *L. seeligeri* RdC, S. *aureus* CECT 5191 and *S*. *epidermidis* CECT 231. *B. breve* DSM 32583 showed a clear inhibitory antimicrobial activity against all indicator organisms used in this study (see **Figure 1**). Subsequently, this strain was screened for production of bacteriocins and/or reuterin but it did not produce these antimicrobial compounds.

### Example 4: Survival of L. fermentum and B. breve DSM 32583 in the presence of oxygen.

Said experiment comprises simulating formula milk preparation with shaking process, which is called "Bottle-Test" (see **Figure 2**). The first step was to mix a simulate formula milk with each strain at the commercial concentration (10⁷ CFU/g). For this purpose, counts in the freeze-dried strains were performed, in triplicate, to assure the concentration of each one at the beginning. Then, based on the previous freeze-dried bacterial counts, a pre-formula was prepared and adjusted to a concentration of 10⁹ CFU/g, using a precision balance. Once the concentration was confirmed, in triplicate, by plate counts in MRScysBP, a final formula milk, for each single strain or for their combination, was prepared at 10⁷ CFU/g concentration. Temperatures and incubation conditions were 37°C in anaerobiosis for 48h; for combinations, 37°C in anaerobiosis for 48h in TOS medium to detect *B*. *breve* DSM 32583 and 37°C in aerobiosis for 48h in MRScys to detect *L. fermentum.* With the bacteria's supplemented formula, baby bottles were prepared by preparing the infant formula according to manufacturer instructions and shaking under aerobic conditions.

### Example 5: Production of riboflavin and folate.

After cultivation in MRS, *B*. *breve* DSM 32583 was washed 3 times with saline solution (0.85 % w/v NaCl), resuspended in this solution at the original culture volume and used to inoculate at 4 % v/v folate, riboflavin or vitamin B12-free culture medium that were then incubated without agitation at 37°C for 18 h. After growth, this washing-resuspension procedure was repeated and the resulting LAB solution was used to inoculate at 2% v/v fresh vitamin-free medium. This last step was repeated 7 times and after the last incubation, samples were taken to determine extra- and intra-cellular vitamin concentrations. A sample (500 µl) of LAB grown vitamin-free medium was mixed with equal parts of protecting buffer for folates (0.1 M phosphate buffer, pH 6.8 containing 1.5% w/v ascorbic acid to prevent vitamin oxidation and degradation) or of a 1% (v/v) acetic acid solution for riboflavin, followed by immediate centrifugation for 5 min at 5000 × *g*. The supernatant was collected (extracellular sample) and the pellet was resuspended in 500 µl of protecting buffer, boiled (100°C) for 5 min, centrifuged for 6 min at 10000 × *g* and the supernanant was collected (intracellular samples). The extracellular samples was also boiled and centrifuged and all supernatants were stored at -70°C until used for vitamin quantification. Folate concentrations were determined using a previously described microbiological assay using *L*. *rhamnosus* NCIMB 10463 as the indicator strain (Laiño et al, 2012). Briefly, samples or different concentrations of HPLC grade folic acid were placed with the indicator strain and incubated statically during 48 h at 37°C in 96 well sterile microplates containing the folate-free medium. The optical density (OD) was read at 580 nm using a microplate reader and the folate concentration of the samples was determined by comparing the OD with those obtained with the standard curve prepared using commercial folic acid. Riboflavin concentrations were determined in the same manner using *L. rhamnosus* ATCC 7469 as the indicator strain grown in the riboflavin-free medium.

### Example 6: CLA and CLNA production.

### Bacterial strains, growth media and conditions:

Eight bifidobacterial strains were used in the study. The bacterial strains were grown overnight at 37°C in MRS broth supplemented with 0.05% (w/v) L-cysteine-HCL (Sigma) and 0.2% (w/v) Tween-80 (MRS-Cys broth) under anaerobic conditions in an anaerobic station. Three percent (v/v) of these cultures were transferred to fresh MRS-Cys broth (10 ml) containing free LA (0.5 mg/ml) and/or free ALA (0.5 mg/ml) and incubated at 37°C for 24 h under anaerobic conditions. The strains that showed CLA production in MRS-Cys broth after an initial qualitative screening were also tested for CLA and/or CLNA production, as described above, in 10% reconstituted skim milk (Scharlau, Sentmenat, Barcelona, Spain) supplemented with 0.05% (w/v) L-cysteine and 0.8% (w/v) casamino acids (milk-based medium).

### Qualitative screening of CLA producers by UV spectroscopy method:

Lipid isolation from culture media was carried out using a chloroform/methanol (2:1, v/v) solution according to Folch method modified by Iverson et al. (2001). The lipid residues obtained were subjected to a N₂ flow and remained dissolved in chloroform at -20°C until spectrophotometric analysis. For this analysis, lipid extracts (200 µl) from each sample were placed on a quartz 96-wells plate and total CLA was quantified at a wavelength of 233 nm in a spectrophotometer according to Rodriguez-Alcalá et al. (2011).

### Quantitative analysis of CLA and CLNA production:

The concentrations of CLA and CLNA in the culture media were determined using a direct methylation method. Heptadecanoic acid (C17:0) was added to the samples as an internal standard. The Fatty Acid Methyl Esters (FAMEs) were dissolved in n-hexane and determined by gas chromatography (GC) in a chromatograph equipped with a VF-23 column (30 m × 0,25 nm × 0,25 µm). For GLC analysis, the initial temperature was 80°C. Then, the temperature was increased to 170°C at 30°C/min, held at 170°C for 3 min, increased to 230°C at 30°C/min and, and finally held at 230°C for 7 min. Helius was used as the carrier gas at a pressure of 15 psig and with a split ratio of 1:50. The injection volume was 0.5 µl and the analysis time was 15 min. Peaks were identified by comparing the retention times of CLA methylated standards and by gas chromatography-mass spectrometry (GC/MS). CLA and CLNA concentrations were expressed as µg/ml and their conversion rates from LA and LNA were calculated using the formula [CLA/(CLA+LA)]×100 and [CLNA/(CLNA+LNA)]×100, respectively.

### Example 7: Total lactate production.

The Lactate production of the selected combinations (*L*. *fermentum, B. breve* DSM 32583, both) was measured with the same strain preparations. Measurement took place at the following time points: 8, 24, 48 h.

Lactate was measured in the samples following the D-lactic acid/L-Lactic acid UV-method kit instructions (r-biopharm/Roche).

Briefly, 1 mL aliquot of each tube was centrifuged for 10 min at 4°C and 16000 x g to separate the phases. After centrifugation, supernatants were diluted with water/puffer/something else based on the initial test performed to adjust the dilution. Diluted samples were analyzed following manufacturer instructions and samples that did not fulfill quality standard (difference in sample absorbance lower than 0.100 at 340 nm) were repeated. After the analysis, lactate values were corrected based on this dilution factor.

### Total lactate results were expressed in mg/L

**Table 1: Total lactate production for each composition comprising B. breve DSM 32583, L. fermentum CECT 5716 or the combination of both of them (1:1).**

| | **Total Lactate (mg/L)** | | |
|---|---|---|---|
| | 8h | 24h | 48h |
| Pre Bio (1:1) | 433.37 | 1394.45 | 2369.79 |
| Pre Bio DSM 32583 | 596.09 | 1514.03 | 1483.35 |
| Pre Bio *L. fermentum* | 221.08 | 349.26 | 493.64 |
| Pre Bio + GOS (1:1) | 825.62 | 2839.90 | 2233.68 |
| Pre Bio + GOS DSM 32583 | 813.31 | 1479.21 | 2146.31 |
| Pre Bio + GOS *L. fermentum* | 594.81 | 814.33 | 723.92 |

### Example 8: Ex-vivo gut model with infant fecal microbiome.

Infant formula with intact or hydrolyzed protein were subjected to a full passage through the oral, gastric and small intestinal phase, the latter involving absorption as described previously (Calatayud, Van den Abbeele et al. 2021). Quantity of protein and other nutrients in both formulas was comparable. Fecal material was collected with parental consent and ethical approval of the University Hospital Ghent (reference number B670201836585) from nine infants (2-8 weeks of age; three born by vaginal delivery and two born by caesarian section; exclusively breastfed). Fecal suspensions were prepared in phosphate buffer, to which reducing agents were added (K2HPO4 8.8 g/L; KH2PO4 6.8 g/L; sodium thioglycolate 0.1 g/L; sodium dithionite 0.015 g/L). The obtained suspensions were mixed with a cryoprotectant (modified version of (Hoefman, Pommerening-Röser et al. 2013) in a 1:1 (v:v) ratio, so that 7.5% fecal suspensions were obtained). The fecal suspensions were flash frozen and then preserved at -80°C (cryostock). Just before the experiment, fecal samples were defrosted and immediately added to the reactors. At the start of the short-term colonic incubation, the test ingredients (probiotic strains) were added to sugar-depleted buffered nutritional medium containing basal nutrients present in the colon. Finally, the cryopreserved fecal suspensions of each of the five donors were added (10% (v:v)). Control conditions had no probiotic addition. Reactors were incubated for 48h at 37°C, under continuous shaking (90 rpm) and anaerobic conditions. The incubations were performed in fully independent reactors with sufficiently high volume (70 mL) in order to not only ensure robust microbial fermentation, but also to allow the collection of multiple samples over time.

### Example 9: SCFA profiles.

The short-chain fatty acids (SCFAs) acetate, butyrate, and propionate (typically occurring in a 3:1:1 ratio) are quantitatively and metabolically the most important microbial end-products of the human colon fermentation process, as they display several physiological effects (Rivière A, Selak M, Lantin D, Leroy F and De Vuyst L (2016) Bifidobacteria and Butyrate-Producing Colon Bacteria: Importance and Strategies for Their Stimulation in the Human Gut. Front. Microbiol. 7:979). Changes in the bacterial ecosystem are associated with changes in the overall metabolic activity. Therefore, changes in metabolites upon probiotic supplementation formula either with intact or hydrolyzed protein was assessed. Supplementation with Bb DSM 32583 or the combination of Bb DSM 32583 +Lf (Lactobacillus fermentum CECT 5716) yielded significantly higher levels of acetate, as well as propionate, compared to unsupplemented (blank) or Lf-supplemented conditions. For acetate and propionate production, all infants showed higher levels upon Bb DSM 32583 supplementation. Overall, Bb and Bb+Lf supplementation showed a trend towards increasing SCFA levels, independent of formula matrix, compared to respective unsupplemented controls (p>0,05). Production of different acids lowered the pH, compared to unsupplemented conditions.

SCFA profiles are optimized by B. breve DSM 32583, independent of food or other probiotics; said effect is likewise visible in the ex vivo system (see Figure 7).

### Example 10: Human moDCs generation.

Human peripheral blood mono nuclear cells (PBMCs) were collected from buffy coats of healthy donors using a Ficoll gradient. CD14⁺ monocytes were isolated from PBMCs by magnetic cell separation (MACS, Miltenyi Biotec, Germany).The obtained monocytes were resuspended at 1 ×10⁶ cells/ml and cultured in RPMI 1640 supplemented with 10% FCS, 25 mM HEPES, and 2 mM glutamine containing 1000 U/ml GM-CSF and 1000 U/ml IL-4. The medium was additionally supplemented with penicillin (100 U/ml) and Streptomycin (100 µg/ml) antibiotics. After 3 days of incubation the medium was replaced by a fresh one containing the above mentioned cytokines and supplements. Immature moDCs cells were harvested at day6 and identified as CD11c+, CD14-, HLA-DR+ cells (AmarY, et al. Immunol Lett. 2015;166(1):6-12).

### Example 11: Stimulation of human moDCs with bacteria or GOS.

Immature moDCs were resuspended at a density of 2 × 10⁵ cells/ml in RPMI-1640 complete medium and seeded in 48-well tissue culture plates. Bacteria (B. *breve* DSM 32583 and *L. fermentum* CECT 5716) were added at different states (live, UV-treated at 15W /5×30min, heat inactivated at 60°C/30min or 80°C/3min) to moDCs and using different ratios of R1=1/1, R2=1/10 and R3=1/100 moDCs to bacteria. GOS was added to moDCs at a concentration range of 0, 0.25, 0.5, 0.75, 1 and 5 mg/ml. Finally, bacteria are added to moDCs in combination with GOS to assess their synbiotic potential. Following an incubation of 48h at 37°C in 5% CO₂, the readout is assessed in terms of maturation marker's expression (CD83, CD86, HLA-DR) and key cytokines production (IL-10, IL-12). The positive control consisted of immature moDC treated with LPS at 100 ng/ml, added in parallel to the other stimuli to immature moDCs.

### Example 12: T cell polarization by moDCs primed with bacteria and GOS.

Naive T cells were purified from Human PBMCs with a CD4⁺ isolation kit (Milteny) then co-cultivated (2×10⁴cells/well) at a ratio of 10/1 with moDCs primed with *B*. *breve* DSM 32583 and *L. fermentum* CECT 5716 as bacteria, GOS or their combination in a round-bottom 96-wells plate. Furthermore, these interactions are assessed in the context of a Th2 polarization induced upon moDCs priming using *Staphylococcus aureus* (*S. aureus*) or IL-33 (25 ng/ml). *S*. *aureus* or IL-33 were added to immature moDCs and the other stimuli (bacteria, GOS) were added alone or in combination with one of them.

At day 6, key cytokines reflecting the Th polarization into Th1, Th2, Th17 and Treg are assessed in culture supernatant, namely: IL-4, IL-5, IL-6, IL-9, IL-10, IL-13, IL-17A, IL-17F, IFN-γ and TNF-α.

### Example 13: Effects of B. Breve DSM 32583 and L. fermentum CECT 5716 on moDCs key inflammatory cytokines.

The tested bacteria and particularly *B*. *breve* DSM 32583 displayed anti-inflammatory signatures when co-cultured with human monocytes derived dendritic cells (moDCs). Indeed, a dose dependent induction of anti-inflammatory IL-10 was observed independently from the physiological state of the bacteria. On the other hand, only a minor upregulation of the pro-inflammatory cytokines IL-12 was detected upon stimulation with *B*. *breve* DSM 32583, resulting in a clear anti-inflammatory profile when considering the IL-10 to IL-12 ratio (see **Figure 8A** and **B**). In conclusion, the prior heat inactivation of the bacteria at 60°C and co-culture with moDCs at an R3 ratio (1/100 cell to bacteria) appear to be the optimum conditions to generate an anti-inflammatory response for both bacteria.

### Example 14: Effects of GOS and IL-33 on moDCs key cytokines and CD83 maturation marker.

The stimulation of human moDCs with galacto-oligosaccharides (GOS) did not lead to any induction of IL-10 nor of IL-12 at the evaluated concentrations nor a surface marker maturation. This indicates that GOS are unable to interact with DCs receptors to induce a detectable immune response (see **Figure 9A**). Likewise, IL-33 did not lead to any perceptible stimulation of cytokine release or CD83 expression at the studied concentrations (see **Figure 9B**). Also, the combined action of GOS or IL-33 with *B*. *breve* did not lead to detectable changes, meaning that *B*. *breve* DSM 32583 (prior heat inactivation of the bacteria at 60°C) induces IL-10 release irrespective of GOS and IL-33 (see **Figure 9C**).

### Example 15: Effects of B. Breve DSM 32583 and L. fermentum CECT 5716 on S. aureus induced Th2 polarization.

Priming of moDCs with both heat-inactivated bacteria, especially *B*. *breve* was able to dampen the *S*. *aureus* induced Th2 response *in vitro,* as reflected by the downregulation of Th2 representative cytokines as IL-13, IL-5 and IL-9. Also, the expression Th1 pro-inflammatory cytokines as IFN-γ, TNF-α and IL-6 was clearly diminished by the action of *B*. *breve,* while with *L. fermentum* a decrease was only seen with IFN-γ (see **Figure 10A**). The Heatmap displays an overview of the cytokines production by Th cells upon co-culture with stimulated moDCs. In addition to the aforementioned cytokines, a slight shift of the IL-4 and IL-17F upon moDCs priming with S. *aureus* and bacteria could be demonstrated (see **Figure 10B**).

### Example 16: Effects of B. Breve DSM 32583 and L. fermentum CECT 5716 on IL-33 induced Th cells polarization.

Upon IL-33 stimulation moDCs transferred inflammatory signals to naive Th cells leading to the expression of both Th2 and Th1 cytokines. A combined priming of moDCs with heat-inactivated B. *breve* and IL-33 leads to a drop of IL-5 and IFN-γ, respective key cytokines of the Th2 and Th1 polarizations (see **Figure 11**).

### Example 17: Effects of B. breve DSM 32583 and L. fermentum CECT 5716 on LPS primed moDCs.

The combined action of heat-inactivated *B*. *breve* with LPS leads to an upregulation of the anti-inflammatory response in moDCs characterized by a synergistic increase in IL-10 and a clear decrease of IL-12 cytokines (see **Figure 12A**) accompanied by a downregulation of CD83, CD86 and HLA-DR maturation markers (see **Figure 12C**). It is likely that the induced IL-10 production in the combined bacteria and LPS action was a determinant factor interfering with the expression of the pro-inflammatory IL-12 cytokine, reducing thereby the expression of surface maturation markers. *L. fermentum,* on the other hand, although displaying anti-inflammatory properties failed to downregulate the IL-12 induces production by moDCs (see **Figure 12B**).

### Example 18: Effects of B. breve DSM 32583 and L. fermentum CECT 5716 on Th cells co-cultured with LPS primed moDCs.

In line with the observations from moDCs co-culture with bacteria, only *B*. *breve* (heat-inactivated) could dampen the Th1 induced inflammation through a significant downregulation of IFN-γ levels (see **Figure 13**).

### Example 19: Effects of living and heat-inactive B. breve DSM 32583 and L. fermentum CECT 5716 on GOS stimulated moDCs.

Priming of moDCs with live and heat-inactivated *B*. *breve* DSM 32583 and *L. fermentum* CECT 5716 leads to an increase of IL-10. Combining GOS with the bacteria results in no effect on the anti-inflammatory effect of *B*. *breve* or *L. fermentum* (see **Figure 14**).

### ITEMS

1. A composition comprising *Bifidobacterium breve* strain, which is deposited with the DSMZ under the accession number DSM 32583, for use in a method of preventing at least one IL-10 mediated disease by enhancing expression of Interleukin 10 (IL-10) in a subject.
2. The composition for the use of item 1, wherein the IL-10 mediated disease is an allergic reaction of the gastrointestinal tract, an allergic reaction of the respiratory tract or an allergic reaction of the skin of said subject.
3. The composition for the use of item 2, wherein said allergic reaction of the gastrointestinal tract comprises food allergy.
4. The composition for the use of item 2, wherein said allergic reaction of the respiratory tract comprises asthma and/or rhinitis.
5. The composition for the use of item 2, wherein said allergic reaction of the skin comprises dermatitis.
6. The composition for the use of item 1, wherein the IL-10 mediated disease is inflammatory bowel disease.
7. The composition for the use of any one of the preceding items, wherein said *Bifidobacterium breve* strain is present in a viable and/or in a non-viable form.
8. The composition for the use of item 7, wherein said *Bifidobacterium breve* strain being present in a viable form is preserved such as spray-dried and/or freeze-dried.
9. The composition for the use of item 8, wherein said *Bifidobacterium breve* strain is present in an amount between about 10³ to about 10¹¹ CFU per gram dry weight of the composition.
10. The composition for the use of item 7, wherein said *Bifidobacterium breve* strain being present in a non-viable form is chemically inactivated and/or mechanically disrupted.
11. The composition for the use of any one of the preceding items, wherein said composition is selected from the group consisting of a nutritional composition, a convenience food product, a nutritional supplement, and a care product.
12. The composition for the use of item 11, wherein said nutritional composition is
   (i) selected from the group consisting of a preterm infant formula, an infant formula, a follow-on infant formula, an enteral nutrition formula, and a growing up milk; or
   (ii) a weaning food.
13. The composition for the use of item 12(i), comprising at least any one of:
   - a total energy content of between about 450 to about 540 kcal/100g based on dry weight of the composition;
   - between about 5 to about 22 wt% (w/w)% of a protein source based on dry weight of the composition;
   - between about 40 to about 66 wt% of a carbohydrate source based on dry weight of the composition;
   - between about 1 to about 12 wt% of a non-digestible oligosaccharide based on dry weight of the composition;
   - between about 20 to about 34 wt% of a fat source based on dry weight of the composition;
   - between about 0.057 to about 0.106 wt% of an ARA source based on dry weight of the composition; or
   - between about 0.041 to about 0.164 wt% of a DHA source based on dry weight of the composition.
14. The composition for the use of any one of the preceding items, comprising a further bacterium, preferably wherein said further bacterium is from the family of Lactobacillaceae or Bifidobacteriaceae.
15. The composition for the use of any one of the preceding items, wherein said subject is at risk of developing an IL-10 mediated disease or is healthy.

## Claims

1. A synthetic composition comprising *Bifidobacterium breve* strain, which is deposited with the DSMZ under the accession number DSM 32583, for use in a method of preventing at least one IL-10 mediated disease by enhancing expression of Interleukin 10 (IL-10) in a subject, wherein the IL-10 mediated disease is an allergic reaction of the skin of said subject.

2. The synthetic composition for the use of claim 2, wherein said allergic reaction of the skin comprises dermatitis.

3. The synthetic composition for the use of any one of the preceding claims, wherein said *Bifidobacterium breve* strain is present in a viable and/or in a non-viable form.

4. The synthetic composition for the use of claim 3, wherein said *Bifidobacterium breve* strain being present in a viable form is preserved such as spray-dried and/or freeze-dried.

5. The synthetic composition for the use of claim 4, wherein said *Bifidobacterium breve* strain is present in an amount between about 10³to about 10¹¹ CFU per gram dry weight of the composition.

6. The synthetic composition for the use of claim 3, wherein said *Bifidobacterium breve* strain being present in a non-viable form is chemically inactivated and/or mechanically disrupted.

7. The synthetic composition for the use of any one of the preceding claims, wherein said synthetic composition is selected from the group consisting of a nutritional composition, a convenience food product, a nutritional supplement, and a care product.

8. The synthetic composition for the use of claim 7, wherein said nutritional composition is
(i) a formula selected from the group consisting of a preterm infant formula, a low birth weight infant formula, an infant formula, a follow-on infant formula, an enteral nutrition formula, and a growing up milk; or
(ii) a weaning food.

9. The synthetic composition for the use of claim 8(i), comprising at least any one of:
- a total energy content of between about 450 to about 540 kcal/100g based on dry weight of the synthetic composition;
- between about 5 to about 22 wt% (w/w)% of a protein source based on dry weight of the synthetic composition;
- between about 40 to about 66 wt% of a carbohydrate source based on dry weight of the synthetic composition;
- between about 1 to about 12 wt% of a non-digestible oligosaccharide based on dry weight of the synthetic composition;
- between about 20 to about 34 wt% of a fat source based on dry weight of the synthetic composition;
- between about 0.057 to about 0.106 wt% of an ARA source based on dry weight of the synthetic composition; or
- between about 0.041 to about 0.164 wt% of a DHA source based on dry weight of the synthetic composition.

10. The synthetic composition for the use of any one of the preceding claims, comprising a further bacterium.

11. The synthetic composition for the use of claim 10, wherein said further bacterium is from the family of Lactobacillaceae or Bifidobacteriaceae.

12. The synthetic composition for the use of any one of the preceding claims, wherein said subject is at risk of developing the IL-10 mediated disease as defined by claim 1 or is healthy.
